# EUROPÄISCHE PATENTSCHRIFT

(11) **EP 2 178 948 B1**
(45) Veröffentlichungstag und Bekanntmachung des Hinweises auf die Patenterteilung: **05.04.2017**
(21) Anmeldenummer: 08787218.0
(22) Anmeldetag: 14.08.2008
(51) Int. Cl.: C08G 77/458, C08G 77/46, C08G 18/77, C08G 18/08

(54) **NEUE POLYHARNSTOFF- UND/ODER POLYURETHAN-POLYORGANOSILOXAN-VERBINDUNGEN**
NOVEL POLYUREA- AND/OR POLYURETHANE-POLYORGANOSILOXANE COMPOUNDS
NOUVEAUX COMPOSÉS POLYORGANOSILOXANES À BASE DE POLYURÉE ET/OU POLYURÉTHANE

(30) Priorität: 14.08.2007 DE 102007038447
(43) Veröffentlichungstag der Anmeldung: 28.04.2010
(73) Patentinhaber: Momentive Performance Materials GmbH, 51368 Leverkusen (DE)
(72) Erfinder: WAGNER, Roland, 53227 Bonn (DE); SOCKEL, Karl-Heinz, 51373 Leverkusen (DE); WITOSSEK, Anita, 40764 Langenfeld (DE); SIMON, Walter, 51375 Leverkusen (DE)
(74) Vertreter: Gille Hrabal
(86) Internationale Anmeldenummer: PCT/EP2008/060681
(87) Internationale Veröffentlichungsnummer: WO 2009/021989

(56) Entgegenhaltungen:
- WO-A-02/077072
- WO-A-2004/104070
- WO-A-2008/113831
- DE-A1- 1 570 576
- US-A- 5 120 813

## Beschreibung

Die Erfindung betrifft neue Polyharnstoff- und/oder Polyurethan-Polyorganosiloxan-Verbindungen, Verfahren zu deren Herstellung, und ihre Verwendung sowie neue reaktive 1- oder 2-Komponenten-Systeme und gehärtete Zusammensetzungen daraus.

Quaternäre Ammoniumstrukturen enthaltende Siloxanblockcopolymere sind umfänglich bekannt. Es kann sich dabei einerseits um Diblockcopolymere vom Typ AB mit Siloxan- und quaternären Ammoniumeinheiten handeln (DE 3340708, EP 282720, US 6240929, US 6730766). Andererseits sind Triblockcopolymere entwickelt worden, die auf der Kombination Siloxan-, quaternären Ammonium- und Polyetherblockeinheiten beruhen (WO 02/10256, WO 02/10257, WO 02/10259, WO 2004/090007, WO 03/078504, WO 2004/041912, WO 2004/042136). Wesentlicher Vorteil dieser Triblockcopolymeren ist, dass deren Struktur flexibel und in sehr weiten Grenzen den konkreten Produktanforderungen angepasst werden können.

Es ist weiterhin bekannt, mit Aminogruppen terminierte Siloxane mit kohlenwasserstoffbasierten Diisocyanaten zu Harnstoffgruppen enthaltenden Diblockcopolymeren umzusetzen (US 2006/036055). Polyethergruppen können nur über eine Harnstoff-Harnstoff oder Urethan-Harnstoff Brücken eingeführt werden. Analoge Urethanderivate sind ebenfalls beschrieben worden (US 2004/087752).

Aus GB 1128642 sind Harnstoff- und Urethangruppierungen enthaltende quaternäre Ammoniumverbindungen bekannt. Die Umsetzung von amino- oder hydroxyterminierten Siloxanen mit Diisocyanaten führt zu isocyanat-terminierten Zwischenstufen, welche dann beispielsweise mit primären-tertiären Di- oder Triaminen reagieren, woraufhin die tertiäre Aminogruppe quaterniert wird. Zwischen Siloxanblock und Quatgruppe befinden in jedem Fall zwei Harnstoffgruppen. Es ist weiterhin möglich, z.B. Oligoethylen-glykole als Kettenverlängerer einzusetzen. Bei deren Einführung werden Isocyanatgruppen verbraucht, welche dann nicht mehr zur Reaktion mit primären-tertiären Di- oder Triaminen zur Verfügung stehen, was zu einer Senkung der Menge an quaternären Ammoniumgruppen führt. Nachteil dieser Lösung ist somit, dass eine flexible, weite Grenzen umfassende Anpassung der Struktur an die konkreten Produktanforderungen nicht erfolgen kann.

Weiterhin ist es bekannt, carbonatfunktionalisierte Siloxane mit primäre und sekundäre Aminogruppen bzw. Hydroxylgruppen enthaltenden Kohlenwasserstoffen zu Urethangruppen enthaltenden Siliconen bzw. entsprechenden Estern umzusetzen (US 5 672 338, US 5 686 547, DE 195 05 892).

Es ist ebenfalls vorgeschlagen worden, ein unsymmetrisch substituiertes Carbonat als Linkergruppe zur Synthese von siloxanmodifizierten diquaternären Verbindungen zu verwenden, welche Urethangruppen enthalten (WO 2005/058863).

Schließlich ist beschrieben worden, diesen unsymmetrisch substituierten Carbonatlinker bei der Synthese von Siloxaneinheiten enthaltenden Polyurethan-Blockcopolymeren mit eingebauten Aminsalzeinheiten zu verwenden (C. Novi, A. Mourran, H. Keul, M. Möller, Macromol. Chem. Phys. 2006, 207, 273-286). Nachteil dieser Verbindungen ist es, dass sie lediglich über pH-sensible Ladungen in Form von Aminsalzen verfügen, was eine reduzierte Substantivität zur Folge hat.

Es ist somit Aufgabe der Erfindung, gegebenenfalls quaternäre Ammoniumgruppen enthaltende Polyharnstoff- und Polyurethan-Polyorganosiloxan-Blockcopolymere zur Verfügung zu stellen, die einerseits eine flexible, weite Grenzen umfassende Anpassung der Struktur an die konkreten Produktanforderungen erlauben und bei denen andererseits unter dem Einfluß von Donor-Akzeptor Wechselwirkungen durch die veränderte Sequenz von Harnstoffgruppen und/oder Urethangruppen Einfluß auf wesentliche Produkteigenschaften genommen werden kann. Weiterhin sollten neuartige Polymerverbindungen mit neuen Eigenschaften bereitgestellt werden, welche bislang noch nicht erschlossene Anwendungsmöglichkeiten zugänglich machen.

Die vorliegende Erfindung stellt neue Polyharnstoff- und/oder Polyurethan-Polyorganosiloxan-Verbindungen zur Verfügung, die aus stabilen Vorstufen im Anwendungsfall gezielt zu den gewünschten Polyharnstoff-und Polyurethan-Polyorganosiloxan-Verbindungen reagieren können. Die neuen Polyharnstoff- und/oder Polyurethan-Polyorganosiloxan-Verbindungen lassen sich einfach, sicher und gezielt herstellen und verfügen über neue interessante Eigenschaften.

Die Erfindung betrifft somit neue Polyharnstoff- und/oder Polyurethan-Polyorganosiloxan-Verbindungen a), enthaltend mindestens ein Strukturelement der Formel (1): 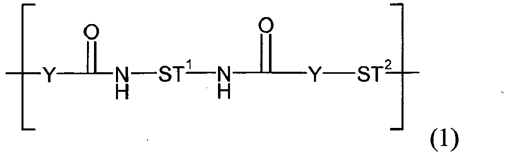 worin
ST¹ und ST² unabhängig voneinander ausgewählt werden aus zwei- oder höherwertigen, geradkettigen, cyclischen oder verzweigten, gesättigten, ungesättigten oder aromatischen, substituierten oder unsubstituierten Kohlenwasserstoffresten mit bis zu 1000 Kohlenstoffatomen (wobei die Kohlenstoffatome einer gegebenenfalls enthaltenen Polyorgano-siloxaneinheit nicht mitgezählt werden), die eine oder mehrere Gruppen, ausgewählt aus -O-,-NH-, NR³-, 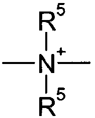 und einer Polydiorganosiloxaneinheit mit 2 bis 1000 Siliciumatomen enthalten können, worin
- R³: ein geradkettiger, cyclischer oder verzweigter, gesättigter, ungesättigter oder aromatischer Kohlen-wasserstoffrest mit bis zu 40 Kohlenstoffatomen ist, der eine oder mehrere Gruppen, ausgewählt aus -O-, -C(O)- und -NH- enthalten kann, und,
- R⁵: ein geradkettiger, cyclischer oder verzweigter, gesättigter, ungesättigter oder aromatischer Kohlenwasserstoffrest mit bis zu 100 Kohlen-stoffatomen ist, der eine oder mehrere Gruppen, ausgewählt aus -O-, -C(O) - und -NH- enthalten kann, oder R⁵ ein zweiwertiger Rest ist, der cyclische Strukturen innerhalb der Reste ST¹ und/oder ST² ausbildet,
und wobei wenn eine Mehrzahl von Resten ST¹ vorliegt, diese gleich oder verschieden voneinander sein können, und wenn eine Mehrzahl von Resten ST² vorliegt, diese gleich oder verschieden voneinander sein können,
mit der Maßgabe, dass
mindestens einer der Reste ST¹ einen Polyorganosiloxanrest umfasst und mindestens einer der Reste ST² einen Polyalkylenoxidrest umfasst, oder
mindestens einer der Reste ST² einen Polyorganosiloxanrest umfasst und mindestens einer der Reste ST¹ einen Polyalkylenoxidrest umfasst.
oder
Polyharnstoff- und/oder Polyurethan-Polyorganosiloxan-Verbindungen b), enthaltend mindestens ein Strukturelement der Formel (3): 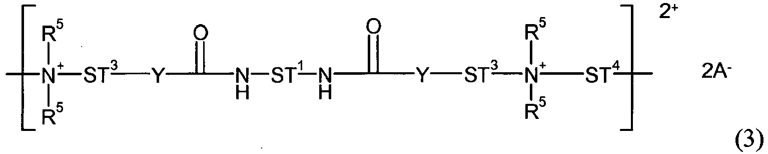 worin
ST¹ ausgewählt wird aus zwei- oder höherwertigen, geradkettigen, cyclischen oder verzweigten, gesättigten, ungesättigten oder aromatischen, substituierten oder unsubstituierten Kohlen-wasserstoffresten mit bis zu 1000 Kohlenstoffatomen (wobei die Kohlenstoffatome einer gegebenenfalls enthaltenen Polyorgano-siloxaneinheit nicht mitgezählt werden), die eine oder mehrere Gruppen, ausgewählt aus -O-, -NH-, -NR³-, 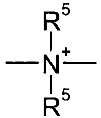 und einer Polydiorganosiloxaneinheit mit 2 bis 1000 Siliciumatomen enthalten können,
worin R³ und R⁵ wie oben definiert sind, und
ST³ ein geradkettiger oder cyclischer oder verzweigter, gesättigter oder ungesättigter oder aromatischer, substituierter oder unsubstituierter Kohlenwasserstoffrest mit 2 bis 100 Kohlenstoffatomen ist, der eine oder mehrere Gruppen -O-, , -NH-, -NR³-, worin R³ wie oben definiert ist, enthalten kann, und
ST⁴ ein geradkettiger oder cyclischer oder verzweigter, gesättigter oder ungesättigter oder aromatischer, substituierter oder unsubstituierter Kohlenwasserstoffrest mit 2 bis 100 Kohlen-stoffatomen (wobei die Kohlenstoffatome einer gegebenenfalls enthaltenen Polyorganosiloxaneinheit nicht mitgezählt werden), der eine oder mehrere Gruppen -O-, , -NH-, -NR³-, worin R³ wie oben definiert ist, und eine Polydiorganosiloxaneinheit mit 2 bis 200 Siliciumatomen enthalten kann, und
A⁻ ein organisches oder anorganisches Anion ist,
mit der Maßgabe, dass mindestens einer der Reste ST¹, ST⁴ einen Polydiorganosiloxan-Rest enthält,
und worin die Polyharnstoff- und/oder Polyurethan-Polyorganosiloxan-Verbindungen b) erhalten werden durch die Umsetzung einer Verbindung der Formel 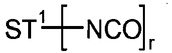 worin ST¹ wie oben definiert ist, mit einer Verbindung der Formel

HY-ST³-NR⁵₂,

worin Y, ST³ und R⁵ wie oben definiert sind,
und anschließend mit einer Verbindung der Formel

Q-ST^{4V}-Q,

umfasst, worin Q ein zur Alkylierung einer Aminogruppe befähigter Rest ist, und ST^{4V} gemeinsam mit dem aus Q nach der Quaternierungsreaktion hervorgehenden Molekülteil den Rest ST⁴ bildet.
Y = -NR²- oder -O- ist, worin
R² Wasserstoff oder ein geradkettiger, cyclischer oder verzweigter, gesättigter, ungesättigter oder aromatischer Kohlenwasserstoffrest mit bis zu 40 Kohlenstoffatomen ist, der eine oder mehrere Gruppen, ausgewählt aus
-O-, -C(O)-, -NH- und NR³- enthalten kann, worin R³ wie oben definiert ist,
oder Säureadditionsverbindungen und oder Salze davon.

Der Begriff Säureadditionsverbindungen meint erfindungsgemäß insbesondere salzartige Verbindungen, die durch Protonierung von basischen Gruppen im Molekül, wie insbesondere gegebenenfalls vorhandenen Aminogruppen, beispielsweise durch Umsetzung mit anorganischen oder organischen Säuren erhalten werden.

Salze der erfindungsgemäßen Verbindungen resultieren insbesondere aus der Bildung quaternäre Ammoniumgruppen-aufweisender Verbindungen, die insbesondere Reste 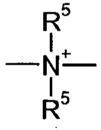 aufweisen.

Erfindungsgemäß ist auch der Fall eingeschlossen, worin R⁵ mit ST⁴ einen stickstoffhaltigen heterocyclischen Rest bilden können.

Substituenten der Kohlenwasserstoffreste für ST¹, ST², ST³ oder ST⁴ schließen bevorzugt ein oder mehrere, bevorzugt ein bis drei Substituenten ein, die bevorzugt ausgewählt werden aus der Gruppe, die besteht aus: Hydroxy, Halogen, wie Fluor oder Chlor und Cyano.

Die vorliegende Erfindung betrifft somit zwei Alternativen a) und b), welche sich insbesondere dadurch unterscheiden, dass die Alternative b) über quaternäre Ammoniumgruppen verfügt, wodurch diese Verbindungen insbesondere über eine hohe Substantivität respektive Haftung und Spreitung auf Oberflächen verfügen. Alternative b) betrifft somit insbesondere Polyharnstoff- und/oder Polyurethan-Polyorganosiloxan-Verbindungen, enthaltend mindestens ein Strukturelement der Formel (3): 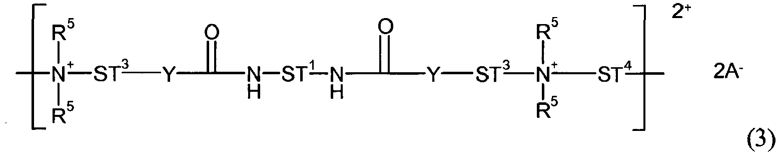 worin
A⁻, R⁵, ST¹, ST³, ST⁴ und Y wie oben definiert sind.

Alternative a) betrifft demgegenüber insbesondere Polyharnstoff- und/oder Polyurethan-Polyorganosiloxan-Verbindungen, enthaltend mindestens ein Strukturelement der Formel (1): 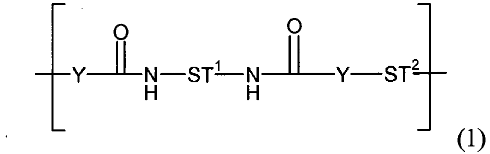 worin
ST¹ und ST² unabhängig voneinander ausgewählt werden aus zwei- oder höherwertigen, geradkettigen, cyclischen oder verzweigten, gesättigten, ungesättigten oder aromatischen, substituierten oder unsubstituierten Kohlenwasserstoffresten mit bis zu 1000 Kohlen-stoffatomen (wobei die Kohlenstoffatome einer gegebenenfalls enthaltenen Polyorganosiloxaneinheit nicht mitgezählt werden), die eine oder mehrere Gruppen, ausgewählt aus -O-, ,-NH-, NR³-, 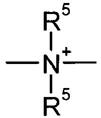 und einer Polydiorganosiloxaneinheit mit 2 bis 1000 Siliciumatomen enthalten können, worin R³ und R⁵ wie oben definiert sind,
und wobei wenn eine Mehrzahl von Resten ST¹ vorliegt, diese gleich oder verschieden voneinander sein können, und wenn eine Mehrzahl von Resten ST² vorliegt, diese gleich oder verschieden voneinander sein können,
mit der Maßgabe, dass
mindestens einer der Reste ST¹ enthält einen Polyorganosiloxanrest und mindestens einer der Reste ST² enthält einen Polyalkylenoxidrest, oder mindestens einer der Reste ST² enthält einen Polyorganosiloxanrest und mindestens einer der Reste ST¹ enthält einen Polyalkylenoxidrest. Es ist aber auch möglich, wenn auch weniger bevorzugt, dass der Rest ST¹ mindestens einen Polyorganosiloxanrest und mindestens einen Polyalkylenoxidrest enthält. Und es ist auch möglich, wenn auch weniger bevorzugt, dass der Rest ST² mindestens einen Polyorganosiloxanrest und mindestens einen Polyalkylenoxidrest enthält.

Der Polyalkylenoxidrest, wie er erfindungsgemäß Anwendung findet, resultiert aus statistischen oder blockartigen Alkylenoxid-Homo- oder Copolymeren, insbesondere auf Basis von Ethylenoxid und/oder Propylenoxid und/oder Butylenoxid. Die Anzahl der Alkylenoxid-Einheiten im Polyalkylenoxidrest liegt beispielsweise bei 2 bis 1000. Besonders bevorzugt sind solche Polyalkylenoxidreste, die auf Ethylenoxid-/ Propylenoxid-Copolymeren beruhen, insbesondere statistische Ethylenoxid-/ Propylenoxid-Copolymere mit 2 bis 100 Ethylenoxid- und Propylenoxid-Einheiten, wobei insbesondere der Anteil der Ethylenoxid-Einheiten größer als der der Propylenoxideinheiten ist.

Besonders bevorzugt sind statistische Copolymere von Ethylenoxid und Propylenoxid der folgenden Formel (4) 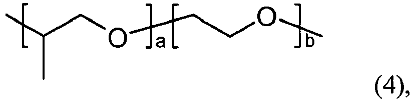 worin a von 0 bis 100 und b von 2 bis 1000 und a+b von 2 bis 1100 sein kann.

Bevorzugt sind diese Reste alkylenterminiert, wie die der Formel (5) 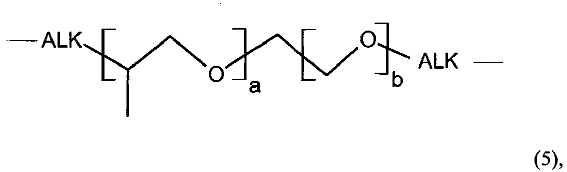 wobei die Reste 'ALK' gleich oder verschieden sein können und aus zweiwertigen geradkettigen oder verzweigten Alkylenresten mit 1 bis 40 Kohlenstoffatomen, die gegenenenfalls substituiert sein können ausgewählt werden, und a und b wie zuvor definiert sind.

In einer bevorzugten Ausführungsform der Erfindung ist ST¹ ein Rest der vorstehenden Formel: 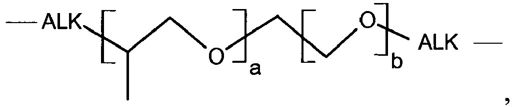 worin 'ALK'und a und b wie oben definiert sind. Die Einführung solcher Reste ST¹ gilt insbesondere durch Verwendung isocyanat-terminierter Polyalkylenoxid-Verbindungen.

In einer weiteren bevorzugten Ausführungsform stellt ST⁴ eine Polyalkylenoxidrestenthaltende Gruppe dar, deren Einführung insbesondere durch Verwendung von halogenalkyl-terminierten Polyalkylenoxid-Verbindungen als Quaternierungsmittel wie unten noch ausführlicher beschrieben gelingt.

D.h., dass in dieser Variante neben mindestens einem hydrophilen Polyalkylenoxidrest mindestens ein hydrophober Polyorganosiloxanrest im Molekül vorliegt. Über das molare Verhältnis dieser Reste bzw. der Kettenlänge der Reste können die Kettenlänge und damit das Molgewicht des Blockco- bzw. Blockterpolymers gesteuert werden. Als Kettenendgruppen bilden sich entweder die reaktiven Gruppen der im Überschuß eingesetzten reaktiven Vorstufe aus. Das sind NCO- OH-, NR₂- und/oder Epoxygruppen oder Umsetzungprodukte von reaktiven Gruppen mit anwesenden oder gezielt zugesetzten wasserstoffaktiven Verbindungen ausgewählt aus monofunktionellen Alkoholen, Thiolen, Aminen, Epoxiden oder Säuren. Das molare Verhältnis und das Molgewicht beeinflussen Eigenschaften, wie insbesondere Oberflächenhaftung, Hydrophilie, Weichheit, Tieftemperaturflexibilität, Spreitungseigenschaften, Gaspermeabilität etc., die somit massgeschneidert werden können.

Die erfindungsgemäßen Polyharnstoff- und/oder Polyurethan-Polyorganosiloxan-Verbindungen enthalten bevorzugt mindestens ein Polydiorganosiloxan-Strukturelement der Formel (6): 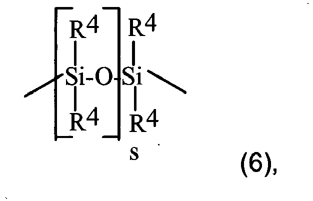 worin
R⁴ ein geradkettiger, cyclischer oder verzweigter, gesättigter, ungesättigter oder aromatischer, substituierter oder unsubstituierter Kohlenwasserstoffrest mit bis zu 20 Kohlenstofffatomen ist, und
s = 1 bis 999 ist.

Bevorzugt ist
- R⁴: C₁ bis C₂₀, bevorzugter C₁ bis C₉, geradkettiger oder cyclischer oder verzweigter, gesättigter oder ungesättigter oder aromatischer Kohlenwasserstoffrest, besonders bevorzugt Methyl und Phenyl und
- s: ist 1 bis 199, bevorzugter 1 bis 99.

Die erfindungsgemäßen Polyharnstoff- und/oder Polyurethan-Polyorganosiloxan-Verbindungen enthalten noch bevorzugter mindestens ein Polydiorganosiloxan-Strukturelement der Formel (6'): 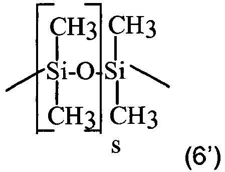 worin s wie zuvor definiert ist.

Die erfindungsgemäßen Polyharnstoff- und/oder Polyurethan-Polyorgano-siloxan-Verbindungen enthalten im Mittel bevorzugt mindestens zwei, bevorzugter mindestens drei Strukturelemente der Formel (1).

Die erfindungsgemäßen Polyharnstoff- und/oder Polyurethan-Polyorgano-siloxan-Verbindungen enthalten bevorzugt im Mittel mindestens zwei, bevorzugt mindestens drei Polydiorganosiloxan-Strukturelemente der Formel (6) oder (6').

In einer bevorzugten Ausführungsform der Erfindung weisen die Polyharnstoff- und/oder Polyurethan-Polyorganosiloxan-Verbindungen zweiwertige Reste ST¹, ST², ST³ und ST⁴ auf, so dass die Polyharnstoff- und/oder Polyurethan-Polyorganosiloxan-Verbindungen linear aufgebaut sind. In bestimmten Anwendungen ist es jedoch bevorzugt, dass durch die Verwendung von mehr als zweiwertigen Resten, insbesondere drei- oder vierwertigen Resten ST¹, ST², ST³ und ST⁴ verzweigte Polyharnstoff- und/oder Polyurethan-Polyorganosiloxan-Verbindungen gebildet werden. Dadurch können elastomere oder duromere und auch hinsichtlich ihrer Lösungsmittelbeständigkeit verbesserte Verbindungen erhalten werden. Bevorzugt werden dabei Verbindungen mit zweiwertigen Resten ST¹, ST², ST³ und ST⁴ und höherwertigen Resten ST¹, ST², ST³ und ST⁴ hergestellt.

In einer weiteren bevorzugten Ausführungsform der Erfindung handelt es sich um reine Polyharnstoff-Polyorganosiloxan-Verbindungen, d.h. Y ist NH oder NR², worin R² wie oben definiert ist. Diese eignen sich besonders gut als Weichmacher und zur Herstellung gaspermeabler Membranen und Tieftemperaturschlagzäh-Modifikatoren.

Die erfindungsgemäßen Polyharnstoff- und/oder Polyurethan-Polyorgano-siloxan-Verbindungen enthalten bevorzugt mindestens einen Rest der Formel: 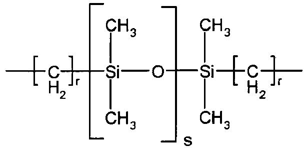 worin s wie oben definiert ist, r von 1 bis 12, bevorzugt 1 oder 3 ist.

Die erfindungsgemäßen Polyharnstoff- und/oder Polyurethan-Polyorganosiloxan-Verbindungen könne bevorzugt nach einem Verfahren erhalten werden, welches die Umsetzung einer Verbindung der Formel 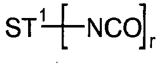 worin ST¹ wie oben definiert ist, und r ≥ 2 ist, mit einer Verbindung der Formel 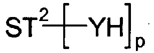 umfasst, worin ST² wie oben definiert ist, und p ≥ 2 ist, mit der Maßgabe, dass
die Polyharnstoff- und/oder Polyurethan-Polyorganosiloxan-Verbin-dungen mindestens einen Polyorganosiloxanrest und mindestens einen Polyalkylenoxidrest umfassen, und insbesondere der Rest ST¹ einen Polyorganosiloxanrest umfasst und der Rest ST² einen Polyalkylenoxidrest umfasst, oder
der Rest ST² einen Polyorganosiloxanrest umfasst und der Rest ST¹ einen Polyalkylenoxidrest umfasst.

In einer bevorzugten Ausführungsform dieses Verfahrens sind r und p = 2, d.h. es werden lineare Polyharnstoff- und/oder Polyurethan-Polyorganosiloxan-Verbindungen gebildet.

Y ist bevorzugt-NR²-, so dass Polyharnstoff-Polyorganosiloxan-Verbindungen gebildet werden.

In dem erfindungsgemäßen Verfahren zur Herstellung der Polyharnstoff- und/oder Polyurethan-Polyorganosiloxan-Verbindungen werden bevorzugt Verbindungen der Formel 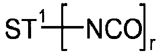 eingesetzt, welche ausgewählt werden aus:
aliphatischen, zwei- oder mehrwertigen, geradkettigen oder verzweigten Polyisocyanaten mit bis zu 15 Kohlenstoffatomen, beispielsweise Hexamethylendiisocyanat, aliphatischen, zwei- oder mehrwertigen, cyclischen Polyisocyanaten mit bis zu 15 Kohlenstoffatomen, wie beispielsweise 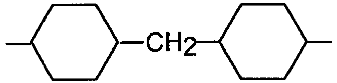 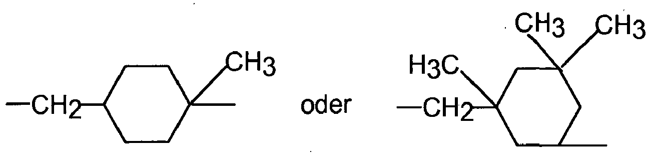
zwei- oder mehrwertige, aromatische Polyisocyanate mit bis zu 15 Kohlenstoffatomen, beispielsweise auf Basis von 2,4-Toluyl, 2,6-Toluyl, Bisphenylmethan- und Naphthylenstrukturen, wie 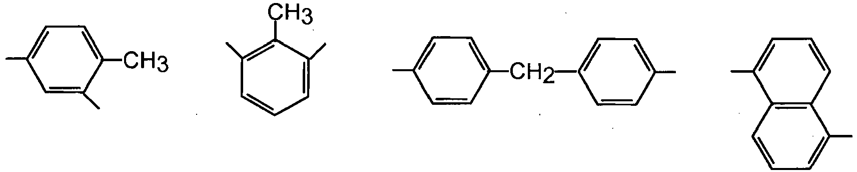 komplexere Polyisocyanate, wie beispielsweise
   isocyanat-terminierte Polyether, bevorzugt ethylenoxid- und propylenoxidbasierte Polyether, beispielsweise hergestellt aus primären aminoterminierten Polyethern vom Typ Jeffamine^{®} , beispielsweise der ED- und T-Serie (Huntsman Corp.),
   isocyanat-terminierte Polyamide,
   isocyanat-terminierte Polyharnstoffe,
   isocyanat-terminierte Polyurethane,
   isocyanatoalkyl-funktionalisierte Polyorganosiloxane, wie höherfunktionelle Polyisocyanate, die sich von primäre Aminogruppen tragenden Polyorganosiloxanen, mit kammartig und gegebenenfalls in α,ω-Position angeordneten primären Aminofunktionen ableiten.

Verbindungen der Formel 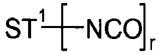 gehen bevorzugt auf primäre Amine zurück und können aus diesen mittels bekannter Synthesemethoden erzeugt werden (Organikum, 17. Auflage, VEB Deutscher Verlag der Wissenschaften, Berlin 1988, S. 427-428). Bevorzugt handelt es sich hierbei um die Umsetzung von primären Aminen oder entsprechenden Aminhydrochloriden mit Phosgen.

R-NH₂ + COCl₂ -> R-N=C=O + 2HCl

Die Isocyanatgruppen, wie sie erfindungsgemäß verwendet werden, können, wie dem Fachmann wohl bekannt ist, blockiert werden. Wann immer in der vorliegenden Erfindung der Begriff "Isocyanat" oder der Rest -NCO erwähnt ist, schließt dies immer auch blockierte Isocyanatgruppen ein, worin die Isocyanatgruppe in an sich bekannter Weise blockiert vorliegt. Auf diese Weise können beispielsweise reaktive 1-Komponenten-Systeme bereitgestellt werden, deren Reaktivität oder Topfzeit ausreichend verringert ist, wie unten noch ausführlicher beschrieben. Die Blockierungsmittel werden bevorzugt ausgewählt aus der Gruppe, die besteht aus substituierten Phenolen, Oximen, Pyrazolen, Dimalonaten, Lactamen Triazolen anderen Ketoestern beispielsweise bevorzugt sind Verbindungen wie Methylethylketoxim, 3,5-Dimethyl-1,2-Pyrazol, Diethylmalonat, ε-Caprolactam, 1,2,4-Triazol oder Ethylacetoacetat. Hiermit kann die Reaktivität in unterschiedlicher Weise auf ein höheres Temperaturniveau angehoben werden, um Topfzeit oder Einbrenntemperatur zu steuern.

Bezüglich Variante a) der erfindungsmäßen Polyharnstoff- und/oder Polyurethan-Polyorganosiloxan-Verbindungen enthält der Rest ST¹ in der Formel 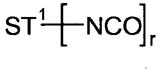 bevorzugt entweder eine Polyalkylenoxidgruppe oder eine Polyorganosiloxangruppe, und der Rest ST² in der Verbindung der Formel 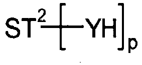 enthält eine Polyalkylenoxidgruppe oder eine Polyorganosiloxangruppe, so dass entweder ST¹ oder ST² eine Polyalkylenoxidgruppe und ST¹ oder ST² eine Polyorganosiloxangruppe enthält.

Besonders bevorzugt enthält ST¹ eine Polyalkylenoxidgruppe und ST² enthält eine Polyorganosiloxangruppe.

Als Verbindung der Formel 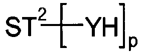 werden bevorzugt solche verwendet, welche ausgewählt werden aus:
aliphatischen, zweiwertigen oder mehrwertigen, geradkettigen oder verzweigten Polyolen oder Polyaminen mit bis zu 15 Kohlenstoffatomen, beispielsweise Hexamethylendiamin oder -diol,
aliphatischen, zweiwertigen oder mehrwertigen, cyclischen Polyolen oder Polyaminen mit bis zu 15 Kohlenstoffatomen, beispielsweise auf Basis von Biscyclohexylmethanstrukturen 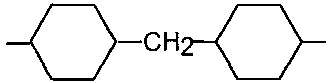 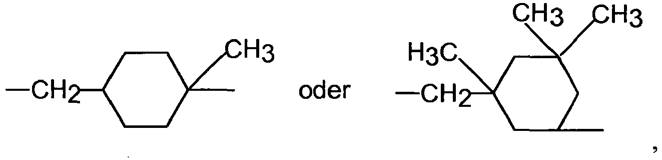
zwei- oder miehrwertigen, aromatischen Polyolen oder Polyaminen mit bis zu 15 Kohlenstoffatomen, beispielsweise auf Basis von 2,4-Toluyl, 2,6-Toluyl, Bisphenylmethan- und Naphthylenstrukturen 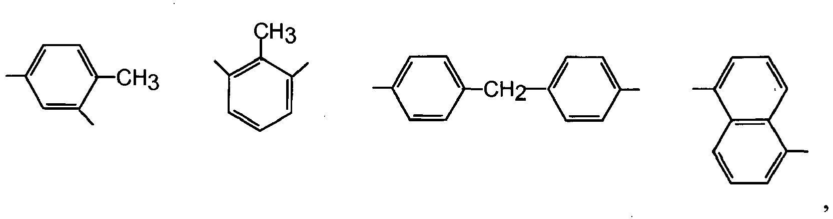 insbesondere Hexamethylendiamin, Phenylendiamine, Toluendiamine, Cyclohexandiamin, Ethylendiamin und Propylendiamin,
zwei- oder höherwertigen primären und/oder sekundären Amine oder Alkoholen, wie beispielsweise N(CH₂CH₂NH₂)₃, Diethylentriamin, Dipropylentriamin und höhere Oligomere, Glycerin und dessen alkoxylierten Derivaten,
komplexere, zwei- oder höherwertigen Polyaminen oder Polyolen, wie primär und sekundär amino- oder hydroxylfunktionalisierte Präpolymere, welche ebenfalls höherfunktionalisiert sein können, wie beispielsweise
   - primär oder sekundär amino- oder hydroxylterminierte Polyether, bevorzugt ethylenoxid- und propylenoxidbasierte Polyether, beispielsweise primär oder sekundär aminoterminierte Polyether vom Typ Jeffamine^{®} , beispielsweise der ED- und T-Serie (Huntsman Corp.)
   - NH₂- terminierte Polyamide,
   - NH₂- terminierte Polyharnstoffe,
   - NH₂- terminierte Polyurethane,
   - OH- terminierte Polyharnstoffe,
   - OH- terminierte Polyurethane,
   - OH- terminierte Polyester,
   - α,ω-primär oder sekundär amino- oder hydroxyalkyl- oder hydroxypolyetheralkylfunktionalisierte Polyorganosiloxane, wie höherfunktionelle primäre oder sekundäre Amino- oder Hydroxyalkyl- oder Hydroxypolyetheralkylgruppen tragende Polyorganosiloxane die über kammartig und gegebenenfalls in α,ω-Position angeordnete Amino- oder Hydroxylfunktionen verfügen.

Besonders bevorzugt werden Verbindungen 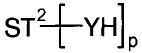 verwendet, die entweder einen Polyorganosiloxanrest oder einen Polyalkyloxidrest, jeweils wie oben beschrieben enthalten.

Die Polyharnstoff- und/oder Polyurethan-Polyorganosiloxan-Verbindungen gemäß Variante b) also insbesondere die der Formel (3) 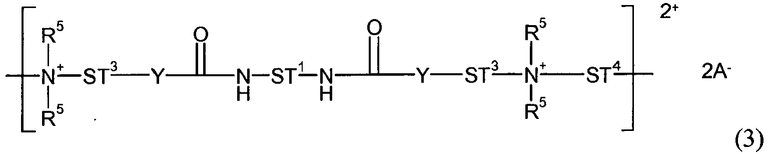 worin
A⁻, R⁵, ST¹, ST³, ST⁴ und Y wie oben definiert sind.
werden bevorzugt durch ein Verfahren erhalten, welches die Umsetzung einer Verbindung der Formel 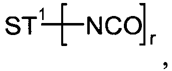 worin ST¹ wie oben definiert ist, mit einer Verbindung der Formel

HY-ST³-NR⁵₂,

worin Y, ST³ und R⁵ wie oben definiert sind,
und anschließend die Umsetzung des erhaltenen Reaktionsproduktes mit einer Verbindung der Formel

Q-ST^{4V}-Q,

umfasst, worin Q ein zur Alkylierung einer Aminogruppe befähigter Rest ist, und ST^{4V} gemeinsam mit dem aus Q nach der Quaternierungsreaktion hervorgehenden Molekülteil den Rest ST⁴ bildet.

Die dabei verwendeten Verbindungen der Formel:

HY-ST³-NR⁵₂,

werden bevorzugt ausgewählt aus:
primär-tertiären oder sekundär-tertiären Diaminostrukturen, wie beispiels-weise N,N-Dimethylpropylendiamin und N-Methylpiperazin,
primär-sekundären Diaminen, beispielsweise Aminoethylethanolamin, oder höherfunktionellen Aminen, wie N,N,N',N'-Tetramethyldipropylentriamin und N,N,N',N'-Tetramethyldiethylentriamin oder
tertiär aminofunktionalisierten Alkoholen, z.B. HOCH₂CH₂N(CH₃)₂.

Die zur Quaternierung respektive Alkylierung der Aminogruppen befähigten Reste Q werden bevorzugt ausgewählt aus Epoxygruppen, Halogencarbonsäureestergruppen und Halogenalkylgruppen. Zur Illustrierung des Restes ST^{4V} dient folgendes Beispiel: 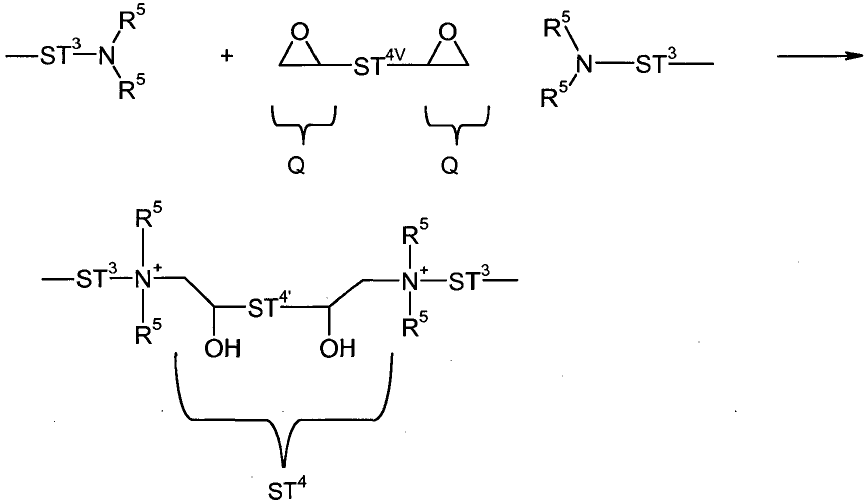 ST⁴ wird also aus ST^{4V} und aus dem aus den Epoxygruppen resultierenden Molekülteilen gebildet.

Die als Quaternierungsmittel verwendeten Verbindungen der Formel

Q-ST^{4V}-Q,

werden bevorzugt ausgewählt aus:
zwei- oder gegebenenfalls auch höherwertigen Epoxygruppen-, Halogencarbonsäureestergruppen- oder Halogenalkylgruppen enthaltenden Alkylierungsmitteln, besonders bevorzugt kohlenwasser-stoffbasierten α,ω-epoxy- oder halogenfunktionalisierten Substanzen, speziell
   - Kohlenwasserstoffdiepoxiden,
      ∘ beispielsweise Vinylcyclohexendiepoxid
      ∘ Epichlorhydrin
      ∘ epoxyterminierte Polyether, bevorzugt ethylenoxid- und propylenoxidbasierte Polyether, beispielsweise glycidyltermininierten Polyether,
      ∘ epoxyterminierte Polyester,
      ∘ epoxyterminierte Polycarbonate,
   - halogenfunktionalisierten Kohlenwasserstoffderivaten, bevorzugt Chloriden und Bromiden,
      ∘ beispielsweise Kohlenwasserstoffdihalogenide,
      ∘ halogenterminierte Polyether, bevorzugt ethylenoxid- und propylenoxidbasierte Polyether,
   - Halogenalkylcarbonsäureestern erhältlich aus Kohlenwasser-stoffdiolen und Polyethern, bevorzugt ethylenoxid- und propylenoxidbasierten Polyethern, speziell Chloressigsäureester, Chlorpropionsäureester und Chlorbutansäureester von Kohlenwasserstoffdiolen und Polyethern,
   - in entsprechende Glycidyl-, Halogen oder Halogenalkylcarbonsäureesterderivate überführten difunktionellen Säurealkoxylaten, beispielsweise Bernsteinsäurealkoxyderivaten,
   - komplexere α,ω-epoxy- oder halogenterminierte Verbindungen, die sich von α,ω-hydroxyfunktionalisierten Präpolymeren ableiten, bevorzugt den Umsetzungsprodukten von Diolen mit Diisocyanaten, OH-terminierten Polyethern, bevorzugt ethylenoxid- und propylenoxidbasierten Polyethern mit Diisocyanaten, OH-terminierten Polyestern, OH-terminierten Polycarbonaten, welche in die entsprechenden α,ω- Halogencarbonsäureester, speziell Chloressigsäureester, Chlor-propionsäureester und Chlorbutansäureester überführt werden,
   - Polyorganosiloxanblöcke enthaltende Verbindungen, bevorzugt α,ω-epoxyterminierte Polyorganosiloxane, bevorzugt α,ω-glycidyl- und epoxycyclohexylterminierte Polyorganosiloxane, α,ω-halogenalkyl-terminierte Polyorganosiloxane, bevorzugt chlorpropyl- und chlorpropenyl-terminierte Polyorganosiloxane, α,ω-halogencarbonsäure-esterterminierte Polyorganosiloxane, bevorzugt Ester der Chloressigsäure, Chlorpropionsäure und Chlorbutansäure, α,ω-halogencarbonsäureester-terminierte Polyetherpolyorganosiloxane, bevorzugt Ester der Chloressigsäure, Chlorpropionsäure und Chlorbutansäure, α,ω-epoxy- oder halogenfunktionalisierte Polyorganosiloxanpräpolymeren, welche bevorzugt aus den entsprechenden α,ω-hydroxyalkyl oder α,ω-hydroxpolyetherterminierten Siloxanpräpolymeren gewonnen werden können,
   - höherfunktionellen kohlenwasserstoffbasierten oder siloxan-basierten Substanzen mit mehr als zwei der vorstehend behandelten Epoxy-oder Halogenfunktionen, beispielsweise den Glycidyl- oder ChloressigsäureesterDerivaten von Glycerol, Pentaerythrol, Sorbitol, und von deren Ethoxylaten/Propoxylaten,
   - Glycidyl- oder Chloressigsäureesterderivaten höherfunktioneller Säurealkoxylate, beispielsweise abgeleitet von Trimellitsäure oder Pyromellitsäure, und
höherfunktionellen siloxanbasierten Substanzen mit α,ω- und/oder kammartiger Epoxy-oder Halogen, bevorzugt Halogencarbonsäure-estersubstitution, wobei die Funktionalität dieser höherfunktionellen kohlenwasserstoffbasierten oder siloxanbasierten Substanzen größer 2 ist.
Die Einführung von Siloxanblöcken in die Einheit ST² erfolgt beispielsweise ausgehend von α,ω-primär oder sekundär amino- oder hydroxyalkyl- oder hydroxypolyetheralkylfunktionalisierten Polyorganosiloxanen. Die Herstellung der entsprechenden α,ω-primär oder sekundär aminoalkyl- oder hydroxyalkyl- oder hydroxypolyetheralkyl-funktionalisierten Polyorganosiloxane ist Stand der Technik (Silicone, Chemie und Technologie, Vulkan Verlag Essen 1989, S. 85-90).
Weiterhin können α,ω-primär oder sekundär aminoalkyl- oder hydroxyalkyl- oder hydroxypolyetheralkyl-funktionalisierte Präpolymere eingesetzt werden, die bevorzugt durch Reaktion von α,ω-primär oder sekundär aminoalkyl-terminierten Siloxanen mit Diisocyanaten, α,ω-hydroxyalkyl-terminierten Siloxanen mit Diisocyanaten, α,ω-hydroxylpolyether-terminierten Siloxanen mit Diisocyanaten zugänglich sind.

Es liegt weiterhin im Rahmen der Erfindung, höherfunktionelle primäre oder sekundäre Aminogruppen oder Hydroxyalkyl- oder Hydroxypolyetheralkyl-Gruppen tragende Polyorganosiloxane zur Formierung des Kohlenwasserstoffrestes ST² einzusetzen. Diese kammartig und gegebenfalls α,ω-funktionalisierten Siloxane sind ebenfalls aus dem Stand der Technik bekannt.

Für den Fall quaternierter Polyharnstoff- und/oder Polyurethan-Polyorgano-siloxan-Verbindungen, werden die isocyanat-terminierten ST¹-Intermediate zunächst mit Verbindungen umgesetzt, die das Element ST³ enthalten. Anschließend erfolgt die Quaternierung unter Einführung des Elementes ST⁴.

Bei den ST³ enthaltenden Reaktionspartnern handelt es sich bevorzugt um Substanzen, die über primär-tertiäre oder sekundär-tertiäre Diaminostrukturen verfügen, wie beispielsweise N,N-Dimethylpropylendiamin. Die ebenfalls bevorzugte Verwendung von z.B. N-Methylpiperazin beinhaltet die Möglichkeit, dass sich cyclische ST-Strukturen bilden können. Alternativ können tertiär aminofunktionalisierte Alkohole, z.B. HOCH₂CH₂N(CH₃)₂ zur Einführung von ST³ benutzt werden.

Die Verwendung derartiger ST³-Einheiten mit Diaminostrukturen setzt die gleichzeitige Verwendung von poly-, insbesondere difunktionellen Quaternierungsmitteln voraus, die ST⁴ einbringen. Anderenfalls ist keine Kettenbildung möglich.

Auf diese Weise erfolgt die Bildung der ST²-Strukturelemente der Formel:

-ST³-N⁺(R⁵)₂-ST⁴-N⁺(R⁵)₂-ST³-

Auch die Verwendung von primär-sekundären Diaminen zur Einführung von ST³ ist möglich.

Verbliebene Aminogruppen können dann im Anschluss an den Kettenaufbau gegebenenfalls alkyliert werden.

Beispiele für höherfunktioneller Amine zur Erzeugung verzweigter ST³-Strukturen sind:
N,N,N',N'-Tetramethyldipropylentriamin (Jeffcat ZR50B Huntsman)
N,N,N',N'-Tetramethyldiethylentriamin.

Bei den die ST⁴-Einheiten bildenden Reaktionspartnern handelt es sich um poly-, insbesondere difunktionelle Alkylierungsmittel, die bevorzugt über Epoxygruppen, Halogencarbonsäureestergruppen und Halogenalkylgruppen verfügen.

Bei den ST⁴ enthaltenden Partnern handelt es sich in einer bevorzugten Aus-führungsform um kohlenwasserstoffbasierte α,ω-epoxy- oder halogen-funktionalisierte Substanzen.

Es liegt ebenfalls im Rahmen der Erfindung, difunktionelle Säurealkoxylate in entsprechende Glycidyl-, Halogen oder Halogencarbonsäureesterderivate zu überführen und erfindungsgemäß einzusetzen. Diese leiten sich zum Beispiel von Bernsteinsäure ab.

Die Synthese der besonders bevorzugten Chlorcarbonsäureester erfolgt in bekannter Weise (Organikum, Organisch-Chemisches Grundpraktikum, 17. Auflage, VEB Deutscher Verlag der Wissenschaften, Berlin 1988, S. 402-408) durch Reaktion der Diolkomponente mit den entsprechenden Halogen-carbonsäureanhydriden oder Halogencarbonsäurechloriden.

In einer weiteren Ausführungsform handelt es sich bei den Kohlenwasserstoffresten ST⁴ um komplexere α,ω-epoxy- oder halogen-terminierte Strukturen, die sich von α,ω-hydroxy-funktionalisierten Präpolymeren ableiten. Bevorzugt handelt es sich bei diesen α,ω-hydroxy-funktionalisierten Präpolymeren um die Umsetzungsprodukte von
- Diolen mit Diisocyanaten
- OH terminierten Polyethern, bevorzugt ethylenoxid- und propylenoxidbasierten Polyethern mit Diisocyanaten,
- OH terminierten Polyestern
- OH terminierten Polycarbonaten
Diese α,ω-hydroxyfunktionalisierten Präpolymeren werden in einer bevorzugten Ausführungsform in die entsprechenden α,ω- Halogencarbon-säureester, speziell Chloressigsäureester, Chlorpropionsäureester und Chlor-butansäureester überführt.

Die erfindungsgemäße Einführung von Siloxanblöcken in ST⁴ erfolgt bevorzugt über
- α,ω-epoxyterminierte Siloxane, bevorzugt α,ω-glycidyl- und epoxycyclohexylterminierte Siloxane,
- α,ω-halogenalkylterminierte Siloxane, bevorzugt chlorpropyl- und chlorpropenylterminierte Siloxane
- α,ω-halogencarbonsäureesterterminierte Siloxane, bevorzugt Ester der Chloressigsäure, Chlorpropionsäure und Chlorbutansäure,
- α,ω-halogencarbonsäureesterterminierte Polyethersiloxane, bevor-zugt Ester der Chloressigsäure, Chlorpropionsäure und Chlor-butansäure.

Die Herstellung der in ST⁴ eingehenden α,ω-epoxyterminierten Siloxane und α,ω-halogenalkylterminierten Siloxane ist im Stand der Technik beschrieben (Silicone, Chemie und Technologie, Vulkan Verlag Essen 1989, S. 85-90 und 120).

Die Herstellung von α,ω-halogencarbonsäureester-terminierten Siloxanen kann in Analogie zur Vorgehensweise gemäß WO 02/10256, Beispiel 1 erfolgen. Hierbei werden SiH Siloxane mit Halogencarbonsäureestern von olefinisch oder acetylenisch ungesättigten Alkoholen umgesetzt.

Die Herstellung α,ω-halogencarbonsäureester-terminierten Polyethersiloxanen kann in Analogie zu WO 02/10257 Beispiel 1 erfolgen. Hierbei werden SiH Siloxane mit Halogencarbonsäureestern olefinisch oder acetylenisch ungesättigter Polyether umgesetzt. Alternativ ist es möglich, Polyether-siloxane mit Halogencarbonsäuren, deren Anhydriden oder Säurechloriden umzusetzen (US 5 153 294, US 5 166 297).

In einer weiteren Ausführungsform erfolgt die Einführung von Siloxanblöcken in ST⁴ über α,ω-epoxy- oder halogenfunktionalisierte Siloxanpräpolymere, welche bevorzugt aus den entsprechenden α,ω-hydroxyalkyl oder α,ω-hydro-xypolyether-terminierten Siloxanpräpolymeren gewonnen werden können.
Diese OH-terminierten siloxanhaltigen Präpolymeren werden bevorzugt durch Reaktion von
- α,ω-hydroxyalkyl-terminierten Siloxanen mit Diisocyanaten,
- □ α,ω-polyether-terminierten Siloxanen mit Diisocyanaten
hergestellt und anschließend in die Epoxy- und Halogenderivate überführt. Eine bevorzugte Ausführungsform stellen die α,ω- halogencarbonsäure-funktionalisierten Siloxanpräpolymere dar, welche durch Veresterung mit z.B. den Anhydriden und Säurechloriden zugänglich sind. Es liegt weiterhin im Rahmen der Erfindung, höherfunktionelle kohlenwasserstoffbasierte oder siloxanbasierte Substanzen zur Formierung des Restes ST⁴ einzusetzen. Diese Materialien enthalten mehr als zwei der vorstehend behandelten Epoxy-oder Halogenfunktionen.

Beispiele für höherfunktionelle kohlenwasserstoffbasierte Substanzen sind die Glycidyl- oder Chloressigsäureester-Derivate von Glycerol, Pentaerythrol, Sorbitol, und von deren Ethoxylaten/Propoxylaten. Es liegt ebenfalls im Rahmen der Erfindung, höherfunktionelle Säurealkoxylate in entsprechende Glycidyl- oder Chloressigsäureester-Derivate zu überführen und erfindungs-gemäß einzusetzen. Diese leiten sich zum Beispiel von Trimellitsäure oder Pyromellitsäure ab.

Geeignete höherfunktionelle siloxanbasierte Substanzen mit α,ω- und/oder kamm-artiger Epoxy- oder Halogen, bevorzugt Halogencarbonsäureester-Substitution können beispielsweise aus hydroxyfunktionellen Precursoren gewonnen werden, welche durch Addition von Allylakohol, Butindiol und den Alkoxylaten von Allylakohol oder Butindiol an entsprechende SiH- Siloxane zugänglich werden. Alternativ können beispielsweise ungesättigte epoxy- oder halogencarbonsäureester-funktionelle Vorstufen an entsprechende SiH Siloxane addiert werden.
Wesentlich ist, dass die Funktionalität dieser höherfunktionellen kohlenwasserstoffbasierten oder siloxanbasierten Substanzen größer 2 ist.Es liegt weiterhin im Rahmen der Erfindung, monofunktionelle kohlenwasser-stoffbasierte oder siloxanbasierte Substanzen zur Formierung des Restes ST⁴ einzusetzen. Diese Materialien enthalten eine der vorstehend behandelten Epoxy-oder Halogenfunktionen.
Beispiele für monofunktionelle kohlenwasserstoffbasierte Substanzen sind die Glycidyl- oder Chloressigsäureesterderivate von Alkanolen, beispielsweise Ethanol, 2-Propanol, Dodecanol und Octadecanol, Alkenolen, beispielsweise Allylalkohol, Hexenol, Oleylalkohol und Alkinolen, beispielsweise Propinol, und die Alkoxylate, speziell Ethoxylate/Propoxylate der genannten mono-funktionellen Alkohole. Es liegt ebenfalls im Rahmen der Erfindung, Fettsäurealkoxylate in entsprechende Glycidyl- oder Chloressigsäureester-Derivate zu überführen und erfindungsgemäß einzusetzen. Geeignete monofunktionelle siloxanbasierte Substanzen mit Epoxy- oder Halogen-, bevorzugt Halogencarbonsäureestersubstitution sind z.B. aus WO 02/10256 bekannt. Sie können beispielsweise aus ungesättigten epoxy- oder halogencarbonsäureesterfunktionellen Precursoren gewonnen werden, welche an entsprechende SiH-Siloxane addiert werden.
Dem Wesen nach werden diese monofunktionellen kohlenwasserstoffbasierten oder siloxanbasierten ST⁴-Vorstufen zugesetzt, um das Molekulargewicht der gebildeten Polymeren zu regulieren und gegebenenfalls in Kooperation mit den höherfunktionellen ST⁴-Precursoren den Verzweigungsgrad der Polymerketten zu steuern.

Bei Verwendung von epoxyhaltigen Substanzen zur Einführung von ST⁴-Gruppen wird in aus dem Stand der Technik bekannter Weise in stöchiometrischen Mengen Säure zugesetzt. Bei den Anionen handelt es sich um anorganische Anionen, wie Halogenid, speziell Chlorid und organische Anionen, wie Carboxylat, speziell C₂ bis C₁₈-Carboxylat, Alkylpolyether-carboxylat, Alkylsulfat, speziell Methosulfat, Sulfonat, speziell Alkylsulfonat und Alkylarylsulfonat, ganz speziell Toluylsulfonat.

Im Ergebnis der dargestellten Gesamtreaktionssequenz werden quaternierte oder nicht quaternierte Polyharnstoff- und/oder Polyurethan-Polyorganosiloxan-Verbindungen erhalten, die mindestens in einem der Strukturelemente ST¹ und/oder ST² über Siloxaneinheiten verfügen.

Die Reaktionen von Diisocyanate mit den amino- oder hydroxylfunktionellen ST²-, ST³- und ST⁴-Precursoren werden bevorzugt im Bereich von Raumtemperatur bis 160 °C, bevorzugt bis 140 °C ausgeführt. Die Reaktionszeiten betragen wenige Minuten bis einige Stunden.

Es liegt im Rahmen der Erfindung, die gesamte Reaktionssequenz oder einzelne Teilschritte ohne Lösemittel oder aber in Gegenwart von Lösemitteln auszuführen. Bevorzugte Lösemittel sind typische Lacklösemittel, wie Methoxy-propylacetat, Butylacetat oder Toluen.

Die vorliegende Erfindung stellt nicht nur neue Polyharnstoff- und/oder Polyurethan-Polyorganosiloxan-Verbindungen sondern insbesondere auch reaktive 1- oder 2-Komponentensysteme bereit, die zu deren Bildung geeignet sind. Diese Systeme enthalten insbesondere mindestens eine Verbindung der Formel 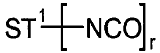 worin ST¹ wie oben definiert ist, und r ≥ 2 ist, worin die Isocyanatgruppen gegebenenfalls in an sich bekannter Weise blockiert sein können,
und mindestens eine Verbindung der Formel 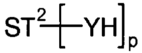 worin ST² wie oben definiert ist, und p ≥ 2 ist, mit der Maßgabe, dass
der Rest ST¹ einen Polyorganosiloxanrest umfasst und der Rest ST² einen Polyalkylenoxidrest umfasst, oder
der Rest ST² einen Polyorganosiloxanrest umfasst und der Rest ST¹ einen Polyalkylenoxidrest umfasst,
sowie gegebenenfalls Füllstoffe, Pigmente, weitere Bindemittel, Hilfsstoffe, Lösungsmittel.

Wie zuvor bereits dargelegt, kann es sich bei den Verbindungen der Formel 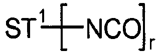 um sowohl freie Isocyanat-Verbindungen, welche insbesondere in 2-K-Systemen Anwendung finden, oder um blockierte Isocyanat-Verbindungen, welche insbesondere in 1-K-Systemen Anwendung finden können.

Die Erfindung betrifft somit weiterhin gehärtete Zusammensetzungen, die aus den reaktiven Systemen, insbesondere den reaktiven 1- oder 2-Komponen-ten-Systems durch Härten, insbesondere bei erhöhten Temperaturen von beispielsweise mehr als 60 °C bevorzugter mehr als 100 °C erhältlich sind.

Ein weiteres reaktives 1- oder 2-Komponentensystem, bei deren Härtung es zur Bildung von Polyharnstoff- und/oder Polyurethan-Polyorganosiloxan-Verbindungen gemäß Variante b), welche über diquaternäre Wieder-holungseinheiten verfügen, kommt, enthält mindestens ein Umsetzungsprodukt aus mindestens einer Verbindung der Formel 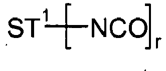 worin ST¹ wie oben definiert ist, und die -NCO-Gruppen blockiert sein können, und mindestens eine Verbindung der Formel

HY-ST³-NR⁵₂,

worin Y, ST³ und R⁵ wie oben definiert sind,
und mindestens eine Verbindung der Formel

Q-ST^{4V}-Q,

worin Q ein zur Alkylierung einer Aminogruppe befähigter Rest ist, und ST^{4V} gemeinsam mit dem aus Q nach der Quaternierungsreaktion hervorgehenden Molekülteil den Rest ST⁴ bildet,
sowie gegebenenfalls Füllstoffe, Pigmente, weitere Bindemittel, Hilfsstoffe, Lösungsmittel.

Die Erfindung betrifft somit auch gehärtete Zusammensetzungen, welche durch Härten dieses reaktiven 1- oder 2-Komponentensystems erhältlich sind.
Die Erfindung betrifft weiterhin auch die Verwendung der erfindungsgemäßen Polyharnstoff- und/oder Polyurethan-Polyorganosiloxan-Verbindungen oder der erfindungsgemäßen reaktiven 1- oder 2-Komponentensysteme oder der daraus erhaltenen gehärteten Zusammensetzungen beispielsweise zur Herstellung von Beschichtungen, Mitteln zur Oberflächenmodifizierung, Elastomeren, Duromeren, bzw. Formkörpern daraus, Klebstoffen, wie Heißschmelzkleber, Siegelmaterialien, insbesondere für Folien, bzw. Foliennähten und Reaktivkleber, als Primer für Metall- und Kunststoffoberflächen, Polymeradditiven, Waschmitteladditiven, rheologischen Mitteln, kosmetischen Mitteln, Fasermodifizierungsmitteln.

Bevorzugt finden die erfindungsgemäßen Polyharnstoff- und/oder Poly-urethan-Polyorganosiloxan-Verbindungen oder die erfindungsgemäßen reak-tiven 1- oder 2-Komponenten-Systeme oder die daraus erhaltenen gehärteten Zusammensetzungen Anwendung bei der Herstellung von Beschichtungen oder als, oder in Mitteln zur Oberflächenmodifizierung von harten Ober-flächen, wie beispielsweise von Glas, Keramik, Kacheln, Beton und Metall, insbesondere Stahlteilen, wie Autokarosserien und Schiffskörper, zur Her-stellung von Primern zur Verbindung von Siliconelastomeren mit anderen Substraten, wie Metall, insbesondere Stahl, Aluminium, Glas, Kunststoffen, wie Epoxidharzen, Polyamiden, Polyphenylensulfiden, Polyestern, wie Polyterephthalaten,
zur Herstellung von thermoplastischen Kunststoffe, wie Polyolefine, Poly-amide, Polyurethane, Poly(meth)acrylate, Polycarbonate, Polyester, sei es, dass die thermoplastischen Kunststoffe aus den erfindungsgemäßen Poly-harnstoff- und/oder Polyurethan-Polyorganosiloxan-Verbindungen gebildet werden, oder dass diese als Additive in den Kunststoffen verwendet werden,
zur Herstellung von Tieftemperaturschlagzäh-Modifikatoren, zur Herstellung von Kleb- und Dichtstoffen, zur Herstellung von Elastomeren, von thermo-plastischen Elastomeren, wie beispielsweise zur Herstellung von Kabelumhül-lungen, Schläuchen, Dichtungen, Tastaturmatten, Membranen, wie selektiv gasdurchlässige Membranen, in Beschichtungen, wie Antifouling-, Antihaft-beschichtungen, als gewebeverträgliche Überzüge, in flammgehemmten Überzügen und in biokompatiblen Materialien, in Kosmetika, wie z.B. als Verkapselungsmaterial, in Körperpflegemitteln, als Lackadditive, als Hilfsstoff in Waschmitteln, in Entschäumerformulierungen und in der Textil-bearbeitung, zum Modifizieren von Harzen oder zur Bitumenmodifizierung,
als Verpackungsmaterial für elektronische Bauteile, als Isolations- oder Abschirmungsmaterial, als Dichtungsmaterial in Hohlräumen mit Kondensatwasserbildung, wie in Flugzeugen, Schiffen, Automobilen,
als Additive für Putz-, Reinigungs- oder Pflegemittel, als Additiv für Körperpflegemittel, als Beschichtungsmaterial für Holz, Papier und Pappe,
als Formentrennmittel, als oder in biokompatiblen Materialien in medizinischen Anwendungen wie Kontaktlinsen, als Beschichtungsmaterial für Textilfasern oder textile Gewebe, als Beschichtungsmaterial für Naturstoffe wie z.B. Leder und Pelze, als Material für Membranen und
als Material für photoaktive Systeme z. B. für lithographische Verfahren, optische Datensicherung oder optische Datenübertragung.
Die Erfinung betrifft weiterhin die Verwendung der Polyharnstoff- und/oder Polyurethan-Polyorganosiloxan-Verbindungen, der reaktiven 1- oder 2-Komponenten-Systeme sowie der gehärteten Zusammensetzungen daraus zur Herstellung von Viskositätsreglern, antistatischen Mitteln, von Mischungskomponenten für Silikonkautschuke, die peroxidisch oder durch Hydrosilylierung (Platin-Katalyse) zu Elastomeren vernetzt werden können, und dort zur Modifizierung von Oberflächen-eigenschaften, zur Modifizierung der Diffusion von Gasen, Flüssigkeiten etc. führen, bzw. das Quellverhalten der Silikonelastomere modifizieren, in Weichmachern für Textilfasern, zur Behandlung der Textilfasern vor, während und nach der Wäsche, in Mitteln zur Modifizierung von natürlichen und synthetischen Fasern, wie beispielsweise Haaren, Baumwollfasern und Synthesefasern, wie Polyesterfasern und Polyamidfasern sowie Mischgeweben, von Textilausrüstungsmitteln, in detergenzien-haltigen Formulierungen, wie in auf anionischen, nichtionogenen und/oder kationischen Tensiden beruhenden Waschmitteln und Reinigungsmitteln, speziell in festen oder flüssigen Waschmittelformulierungen in Anteilen von etwa 0,1 bis 10 Gew.-% bezogen auf die Gesamtmenge der Fomulierung, in kosmetischen Formulierungen und Faserbehandlungsformulierungen, wie Textilpflegemitteln, in Anteilen von etwa 0,1 bis 50 Gew.-% bezogen auf die Gesamtmenge der Fomulierung.

Die Erfindung betrifft weiterhin die Verwendung der Polyharnstoff- und/oder Polyurethan-Polyorganosiloxan-Verbindungen oder der reaktiven 1- oder 2-Komponentensysteme sowie den gehärteten Zusammensetzungen daraus zur Behandlung und Ausrüstung von harten Oberflächen, wie Glas, Keramik, Kacheln, Kunststoffoberflächen, Metalloberflächen, Lackoberflächen, speziell Schiffskörpern und Automobilkarosserien, ganz speziell auch in Trocknerformulierungen für die maschinelle Autowäsche, als Kleber bzw. Primer, bevorzugt zur Verbindung von Silikon-elastomeren mit anderen Substraten, wie Stahl, Aluminium, Glas, Epoxidharz, Polyamid, als Modifikatoren, z.B. Tieftemperaturschlagzäh-Modifikatoren und Polaritätsmodifikatoren, für kohlenwasserstoffbasierte Polymere und silikonbasierte Elastomersysteme, die auf peroxidischer und Pt-katalysierter Vernetzung beruhen.

Die Erfindung betrifft weiterhin die Verwendung der Polyharnstoff- und/oder Polyurethan-Polyorganosiloxan-Verbindungen oder der reaktiven 1- oder 2-Komponentensysteme sowie der gehärteten Zusammensetzungen daraus zur Behandlung von natürlichen und synthetischen Fasern, beispielsweise Baumwolle, Wolle, polyester- und polyamidbasierten Synthesefasern, speziell in Form von Textilien, in speziellen Mitteln zur Faserbehandlung, insbesondere in anionische, nichtionische und kationische Tenside enthaltenden Waschmittelformulierungen, wobei die erfindungsgemäßen Verbindungen in das Waschmittel direkt eingebaut sein können, separat in den laufenden Waschprozeß oder nach dem Waschprozeß dosiert werden können, als Bestandteil separater Weichmachersysteme, speziell auf Basis kationischer Tenside, nach der Wäsche von Fasern und Textilien, als Bügelhilfe und Mittel zur Verhinderung bzw. Rückgängigmachung von Textilverknitterungen, zur Faserausrüstung, speziell zur Erstausrüstung und Behandlung von beispielsweise Baumwolle, Wolle, polyester- und polyamidbasierten Synthesefasern, speziell in Form von Textilien, Papier und Holz.

Die Erfindung betrifft weiterhin die Verwendung der Polyharnstoff- und/oder Polyurethan-Polyorganosiloxan-Verbindungen oder der reaktiven 1- oder 2-Komponenten-Systeme sowie der gehärteten Zusammensetzungen daraus in kosmetischen Systemen zur Behandlung von Haaren und Haut, bevorzugt als Reinsubstanz, insbesondere wässrigen bzw. wässrige/organischen Lösungen, Mischungen, Emulsionen oder Mikroemulsion in Form von Flüssigkeiten, Cremes oder Pasten unterschiedlicher Viskosität, in kosmetischen Formulierungen zur Behandlung keratinhaltiger Substrate wie z. B. menschliche und tierische Haare oder Haut, als alkoholische oder polyalkoholische Lösung oder als klare, trübe, weiße Emulsion oder Mikroemulsion, beispielsweise in sogenannten "Rinse-off" Mitteln wie z.B. "2-in-1" Shampoos, "Body Wash"-Formulierungen und Haarspülungen zur Behandlung von Haar während und nach der Reinigung oder nach dem Färben oder der Behandlung von Haar vor der Bleichung, der Lockung oder Entkräuselung, sowie in sogenannten "Leave-in" Mitteln wie Haarkuren, Pflegecremes, Frisiercremes, Haargelen, Haarstylingprodukten, Haarfestigern, Haarsprays, Pumpsprays, Fönwell-mitteln und Fönfestigern, weiterhin in permanenten, semipermanenten und temporären Haarfärbemitteln, in Mitteln zur Erhöhung des Volumens, der Kämmbarkeit und des Glanzes, sowie der Verminderung des Auswaschens der Farbe von und aus gefärbten keratinhaltigen Substraten wie z.B. menschlichem und tierischem Haar.

Bevorzugt finden die erfindungsgemäßen Polyharnstoff- und/oder Poly-urethan-Polyorganosiloxan-Verbindungen oder der reaktiven 1- oder 2-Komponentensysteme sowie den gehärteten Zusammensetzungen daraus in nicht transparenten Weichspülerdispersionen oder Weichspüleremulsionen bzw. transparenten Mikroemulsionen oder Lösungen, in Wäscheauffrischer-Systemen auf festen Trägern. In kosmetischen Formulierungen für Haare können die erfindungsgemäßen Polyharnstoff- und Polyurethan -Polyorganosiloxan-Verbindungen dabei insbesondere die Funktion von sogenannten Konditionierungsmitteln ("conditioner") ausüben, d.h. insbesondere die Haareigenschaften, wie Weichheit, Glanz, Fülle, Kämmbarkeit etc. günstig beeinflussen, wobei sie insbesondere auch in Kombination mit anderen üblichen Konditionierungsmitteln, wie z.B. Poly-alpha-olefine, fluorierte Öle, fluorierte Wachse, fluorierten Kautschuke, Carbonsäureestern mit mindestens 10 Kohlenstoffatomen, kationische Polymeren, in dem Medium der Formulierung unlösliche oder lösliche Silicone, Mineralöle, pflanzliche Ölen und tierische Ölen und deren Gemische, wie beispielsweise in WO 99/009939 beschrieben, verwendet werden können.

Der Ausdruck "zur Herstellung von", wie er vorstehend verwendet wird, schließt dabei auch den Fall ein, dass die erfindungsgemäßen Polyharnstoff- und/oder Polyurethan-Polyorganosiloxan-Verbindungen allein für die genannte Anwendung verwendet werden. D.h., das beispielsweise die erfindungsgemäßen Polyharnstoff- und/oder Polyurethan-Polyorganosiloxan-Verbindungen selbst unmittelbar als Tieftemperaturschlagzähmodifikatoren verwendet werden können. Sie können aber auch vorher etwa durch Mischen, Compoundierung, Masterbatch-Herstellung geeignet gewissermaßen als Additiv in den genannten Anwendungen bereitgestellt werden.

Die vorliegende Erfindung betrifft weiterhin neue Waschmittelformulierungen, kosmetische Formulierungen, Faserbehandlungs-formulierungen, die die erfindungsgemäßen Polyharnstoff- und/oder Polyurethan-Polyorganosiloxan-Verbindungen enthalten.

So können die erfindungsgemäßen Polyharnstoff- und/oder Polyurethan-Polyorganosiloxan-Verbindungen beispielsweise in festen oder flüssigen Waschmittelformulierungen in Anteilen von etwa 0,1 bis 10 Gew.-% bezogen auf die Gesamtmenge der Formulierung vorliegen, in kosmetischen Formulierungen und Faserbehandlungsformulierungen, wie Textilpflegemitteln, in Anteilen von etwa 0,1 bis 50 Gew.-% bezogen auf die Gesamtmenge der Fomulierung vorliegen.

Sie können weiterhin zur Behandlung von natürlichen und synthetischen Fasern, beispielsweise Baumwolle, Wolle, polyester- und polyamidbasierten Synthesefasern, speziell in Form von Textilien, in speziellen Mitteln zur Faserbehandlung, insbesondere in anionische, nichtionische und kationische Tenside enthaltenden Waschmittelformulierungen verwendet werden, wobei die erfindungsgemäßen Verbindungen in das Waschmittel direkt eingebaut sein können, separat in den laufenden Waschprozeß oder nach dem Waschprozeß dosiert werden können, und den behandelten Substraten Weichheit, verbesserte Elastizität und verringerte Knitterneigung bei Erhalt einer akzeptablen Hydrophilie verliehen wird.

Sie können ebenfalls als Bestandteil separater Weichmachersysteme, speziell auf Basis kationischer Tenside, nach der Wäsche von Fasern und Textilien, als Bügelhilfe und Mittel zur Verhinderung bzw. Rückgängigmachung von Textilverknitterungen, dienen.

Sie können weiterhin zur Faserausrüstung, speziell zur Erstausrüstung und Behandlung von beispielsweise Baumwolle, Wolle, polyester- und polyamidbasierten Synthesefasern, speziell in Form von Textilien, Papier und Holz genutzt werden.

Sie können weiterhin wie bereits dargelegt vorteilhaft in kosmetischen Systemen zur Behandlung von Haaren und Haut eingesetzt werden.

Besonders bevorzugte Anwendungsgebiete für die erfindungsgemäßen Polyharnstoff- und Polyurethan -Polyorganosiloxan-Verbindungen sind auch, bevorzugt wässrige, Lösungen, Mischungen, Emulsionen und Mikroemulsionen insbesondere als Basis für kosmetische Formulierungen.

Die erfindungsgemäßen Polyharnstoff- und Polyurethan -Polyorganosiloxan-Verbindungen können als Reinsubstanz, Lösung, Mischung, Emulsion oder Mikroemulsion in Form von Flüssigkeiten, Cremes oder Pasten als Einsatzstoff für die Herstellung geeigneter, erfindungsgemäßer kosmetischer Formulierungen unterschiedlicher Viskositäten verwendet werden.

Das Verfahren zur Herstellung von Formulierungen der erfindungsgemäßen Polyharnstoff- und/oder Polyurethan-Polyorganosiloxan-Verbindungen, wie beispielsweise zur Behandlung von Substraten, wie harte oder weiche Substrate, kann beispielsweise aus den folgenden Schritten bestehen:
a) Herstellen einer Vormischung in Form von Lösungen, Mischungen oder Emulsionen mit den erfindungsgemäßen Polyharnstoff- und/oder Polyurethan - Polyorganosiloxan-Verbindungen und
b) Herstellung einer weiteren Mischung unter Verwendung der Vormischung a) sowie Zusatz gegebenenfalls weiterer Tenside, Hilfsstoffe und anderer Additive oder
c) Zusammenfassen der Schritte a) und b), in dem man das Vermischen der Bestandteile mit Rührern, Dissolvern, Knetern, Pumpen, Mischschnecken, Mischdüsen, Nieder- und Hochdruckemulgiuergeräten durchführt.

Die Verfahren werden mit den in der Technik (Ullmann's Enzyklopädie) bekannten Maschinen und Apparaten, wie z. B. jeder Form von Rührern in geeigneten Behältern, Apparaten bzw. Mischgeräten, wie zuvor beschrieben, verwirklicht.

Die direkte Vermischung aller Bestandteile ist möglich. Es wird jedoch die Herstellung einer Vormischung bevorzugt, da sie zur schnelleren und besseren Verteilung führt und zum Teil unumgänglich ist, da die unterschiedlichen Stoffgruppen andernfalls nicht in geeigneter Weise oder nur unter hohem Aufwand miteinander vermischt bzw. emulgiert bzw. dispergiert werden können. Geeignete Vor- bzw. Zwischenmischungen können bevorzugt Mischungen in Form von Lösungen, Pasten, Cremes oder sonstige Formen von Emulsionen oder Dispersionen sein. Besonders bevorzugt ist die Herstellung und Verwendung von Mikroemulsionen mit 10 bis 200 nm mittlerem Partikeldurchmesser in kosmetischen Formulierungen.

Zur Herstellung von Lösungen und Mischungen, die die erfindungsgemäßen Polyharnstoff- und/oder Polyurethan-Polyorganosiloxan-Verbindungen enthalten, eignen sich bevorzugt alkoholische und polyalkoholische Lösemittel sowie deren Mischungen mit Wasser, ölartigen Substanzen und übliche Silikone (u.a Polydimethylsiloxane) sowie binäre und ternäre Mischungen von Lösemitteln und/oder ölartigen Substanzen und/oder Silikonen. Besonders bevorzugte Lösemittel sind hierbei Ethanol, Isopropanol, Ethylenglykol und Ethylenglykolether, Polyethylenglykole und deren Ether, Propylenglykol und Propylenglykolether, Polypropylenglykole und deren Ether und Glycerin und deren Mischungen. Besonders bevorzugte ölartige Substanzen sind Mineralölprodukte sowie Öle pflanzlichen, tierischen und synthetischen Ursprungs und deren Mischungen. Besonders bevorzugte Silikone, die von den erfindungsgemäßen Polyharnstoff- und/oder Polyurethan-Polyorganosiloxan-Verbindungen verschieden sind, wie cyclische und lineare Polydimethylsiloxane und deren Mischungen wie z. B. (nach INCI) Cyclomethicone, Cyclotetrasiloxane, Cyclopentasiloxane, Cyclohexasiloxane, Dimethicone mit einem Viskositätsbereich von 0,65 bis 60.000.000 mPa.s bei 25 °C und Dimethiconol mit einem Viskositätsbereich von 10 bis 60.000.000 mPa.s bei 25 °C.

Bevorzugte Lösungen und Mischungen, die die erfindungsgemäßen Polyharnstoff- und/oder Polyurethan-Polyorganosiloxan-Verbindungen enthalten, weisen die folgende Zusammensetzung in Gew. %, bezogen auf das Gesamtgewicht der Zusammensetzung, auf:

**Lösungen bzw. Mischungen:**

| | |
|---|---|
| 0.1 - 99,9 Gew.% | erfindungsgemäße Polyharnstoff- und/oder Polyurethan-Polyorganosiloxan-Verbindungen |
| 0.1 - 99,9 Gew.% | Lösemittel und/oder Öl und/oder Silikone, und/oder Wasser |

Zusammensetzungen der Emulsionen der erfindungsgemäßen Polyharnstoff- und/oder Polyurethan -Polyorganosiloxan-Verbindungen:

Zur Herstellung der Emulsionen werden allgemein Wasser und nichtionische, kationische und amphotere Tenside sowie Tensidmischungen verwendet. Außerdem können Emulsionen Hilfsstoffe wie z. B. anorganische und organische Säuren, Basen und Puffer, Salze, Verdicker, Stabilisatoren für Emulsionen wie z. B. 'Xanthan Gum', Konservierungsmittel, Schaumstabilisatoren, Entschäumer und Lösemittel wie z. B. Alkohole (Ethanol, Isopropanol, Ethylenglykol, Polyethylenglykol, Propylenglykol, Polypropylenglykol, Glykolether und Glycerin und deren Mischungen) enthalten.

Die erfindungsgemäßen Polyharnstoff- und/oder Polyurethan-Polyorganosiloxan-Verbindungen, die in den Emulsionen verwendet werden, können bei der Herstellung von Emulsionen auch selbst als Emulgator dienen.

Eine bevorzugte Emulsion, die bevorzugt zur Herstellung von kosmetischen Formulierungen verwendet werden kann, besteht beispielsweise aus folgenden Bestandteilen in Gew.-%, bezogen auf die Gesamtmenge der Zusammensetzung:

| | |
|---|---|
| 10 - 50% | erfindungsgemäße Polyharnstoff- und/oder Polyurethan-Polyorganosiloxan-Verbindungen, |
| 1 - 35 % | Tenside, |
| 0 - 10 % | Hilfsstoffe, |
| 0 - 20 % | Lösungsmittel, |
| auf 100 % | ergänzt durch Wasser. |

Mikroemulsionen für kosmetische Formulierungen, die Ausrüstung von Textilien und anderen faserförmigen Substraten oder die Beschichtung harter Oberflächen.

Besonders bevorzugt ist die Herstellung von Mikroemulsionen mit einem hohen Aktivgehalt an erfindungsgemäßen Polyharnstoff- und/oder Polyurethan-Polyorganosiloxan-Verbindungen, da diese neben der Möglichkeit zur Herstellung klarer kosmetischer Formulierungen zusätzlich den Vorteil der prozesstechnisch einfachen Einarbeitung ("Cold Process") in wässerige Formulierungen bieten. Es besteht die Möglichkeit, die erfindungsgemäßen Polyharnstoff- und/oder Polyurethan-Polyorganosiloxan-Verbindungen bei der Herstellung von Mikroemulsionen in Form der weiter oben beschriebenen Lösungen und Mischungen einzusetzen. Ein bevorzugter Aktivgehalt der erfindungsgemäßen Polyharnstoff- und/oder Polyurethan-Polyorganosiloxan-Verbindungen in der Emulsion liegt zwischen 5 und 60 Gew.-%, besonders bevorzugt sind 10-50 Gew.-% bezogen auf die Gesamtmenge der Zusammensetzung.

Eine ganz speziell bevorzugte Mikroemulsion besteht aus folgenden, die Erfindung jedoch nicht limitierenden Bestandteilen in Gew.-% bezogen auf die Gesamtmenge der Mikroemulsion:

| | |
|---|---|
| 20 - 80% | erfindungsgemäße Polyharnstoff- und/oder Polyurethan-Polyorganosiloxan-Verbindungen |
| 0 - 35% | Tenside |
| 0 - 10% | Hilfsstoffe |
| 0 - 20% | Lösungsmittel |
| auf 100 % | ergänzt durch Wasser. |

Ein weiterer Gegenstand der Erfindung ist die Verwendung der mit den erfindungsgemäßen Polyharnstoff- und/oder Polyurethan -Polyorganosiloxan-Verbindungen hergestellten Lösungen, Mischungen bzw. Emulsionen in einer kosmetischen Formulierung.

Diese kosmetischen Formulierungen werden unter Verwendung der zuvor hergestellten Lösungen oder Emulsionen hergestellt, sie können aber auch direkt aus den Einzelbestandteilen erzeugt werden.

### Kosmetische Formulierungen:

### Kosmetische Formulierungen schließen beispielsweise ein:

Sogenannte "Rinse-off" Produkte wie z.B. "2-in-1" Shampoos, "Body Wash" und Haarspülungen zur Behandlung von Haar während und nach der Reinigung oder nach dem Färben oder der Behandlung von Haar vor der Bleichung, der Lockung oder Entkräuselung, sowie sogenannte "Leave-in" Produkte wie Haarkuren, Pflegecremes, Frisiercremes, Haargele, Haarstylingprodukte, Haarfestiger, Haarsprays, Pumpsprays, Fönwellmittel und Fönfestiger. Darüber hinaus umfassen die Formulierungen ebenfalls Haarfärbemittel, die je nach Waschbeständigkeit des erzielten Farb-ergebnisses in 3 Typen - permanente, semipermanente und temporäre Haarfärbemittel - unterschieden werden können. Der Begriff Haare beinhaltet hierbei alle keratinhaltigen Fasern, inbesondere aber menschliches Haar. Die Haarfärbemittel enthalten beispielsweise neben den erfindungsgemäßen Polyharnstoff- und/oder Polyurethan-Polyorganosiloxan-Verbindungen übliche Silikone, Tenside, Hilfsstoffe und Färbemittel. Jeder dieser Inhaltsstoffe kann entweder allein oder in Kombination mit weiteren Inhaltsstoffen verwendet werden und steht für zusätzliche Funktionen in den Formulierungen, die zur Erhöhung des Volumens, der Kämmbarkeit und des Glanzes sowie der Verminderung des Auswaschens der Farbe von und aus gefärbten keratinhaltigen Substraten wie z.B. menschlichem und tierischem Haar dienen und mindestens eine Polyharnstoff- und/oder Polyurethan -Polyorganosilöxan-Verbindungen enthalten.

Die im Zusammenhang mit den kosmetischen Formulierungen erwähnten Abkürzungen werden im INCI (The Cosmetic, Toiletry and Fragrance Association Washington DC) erklärt.

Die neben den erfindungsgemäßen Polyharnstoff- und/oder Polyurethan -Polyorganosiloxan-Verbindungen hierbei umfaßten Silikone, schließen beispielsweise ein: Cyclische, lineare und verzweigte Polydimethylsiloxane mit einer Viskosität von 0.65 - 200.000.000 mPa.s bei 25 °C sowie deren Mischungen wie z.B. Octaorganocyclotetrasiloxane, Octamethylcyclotetrasiloxane, Deca-organocyclopentasiloxane, Dodecaorganocyclohexasiloxane, wobei der organische Rest bevorzugt Methyl bedeutet, wie SF 1173, SF 1202, SF 1217, SF 1204 und SF 1258 von Momentive Performance Materials, Dimethicone wie die Baysilone M Öle (M3 bis M 2.000.000), SE 30, SF 1214, SF 1236, SF 1276 und CF 1251 von Momentive Performance Materials und Dimethiconole wie Baysilone, SiOH-endgestoppte Polyorganosiloxangums von Momentive Performance Materials und DC 1501 und DC 1503 von Dow Corning.
Der Einsatz der oben beschriebenen Polydimethylsiloxane in Form von nicht-ionischen, anionischen und kationischen Emulsionen wie z.B. SM 2169, SM 2785, SM 555, SM 2167 und SM 2112 von Momentive Performance Materials in Kombination mit Emulsionen der erfindungsgemäßen Polyharnstoff - und/oder Polyurethan-Polyorganosiloxan-Verbindungen und/oder der Einsatz von Mischungen und Lösungen der oben beschriebenen Polydimethylsiloxane mit den erfindungsgemäßen Polyharnstoff- und/oder Polyurethan-Polyorganosiloxan-Verbindungen ist hierbei besonders bevorzugt, da sich aus diesen Kombinationen besondere Eigenschaften von Haarpflegeprodukten ableiten lassen, wie das für bisher bekannte amino-funktionelle Silikone bereits umfangreich in der Literatur beschrieben wurde (WO 99/44565, WO 99/44567, WO 99/49836, WO 99/53889, WO 97/12594, US 6,028,031, EP 0811371, WO 98/18443, WO 98/43599 und US 2002-0182161).
Ebenfalls geeignet sind feste Silikone, sogenannte MQ-Harze, wie z.B. SR 1000 von Momentive Performance Materials und deren Lösungen in Lösungsmitteln wie den oben genannten Silikone und aliphatischen Lösungsmitteln wie z.B. Isododekan.
Ebenfalls geeignet sind organofunktionelle Silikone, wie Alkyl-, Aryl-, Arylalkyl-, Phenyl-, Fluoralkyl-, und Polyether-modifizierte Silikone wie die Typen SF 1632, SF 1642, SF 1555, Baysilone CF 1301, Baysilone PK 20, FF 157, SF 1188A, SF 1288 und SF 1388 von Momentive Performance Materials.

### Tenside:

Tenside als Inhaltstoffe kosmetischer Formulierungen werden beschrieben in A. Domsch: Die kosmetischen Präparate, Verlag für Chem. Industrie, 4. Auflage, 1992, im Kosmetikjahrbuch 1995, Verlag für chemische Industrie, 1995, und H. Stache, Tensidtaschenbuch, 2. Auflage, Carl Hanser Verlag, 1981.

### Anionische Tenside:

Exemplarisch jedoch nicht limitierend sind folgende anionische Tenside als Bestandteil der Formulierungen geeignet:
Alkylsulfate, Alkylethersulfate, Alkarylsulfate, Olefinsulfonate, Alkylamidether-sulfate, Acylisethionate, Acylglutamate, Alkylethercarboxy-late, Methyltauride und Tauride, Sarkoside, Sulfosuccinate, Eiweiß-fettsäurekondensate, Alkylphosphate und Alkyletherphosphate. Hierbei können die freien Säuren sowie deren Alkalimetallsalze, Magnesium-, Ammonium- und Mono-, Di- und Triethanolaminsalze verwendet werden. Die Alkyl- und Acylgruppen enthalten typischerweise 8-18 C-Atome und können ungesättigt sein. Die Alkylthersulfate, Alkylamidethersulfate, Alkylethercarboxylate und Alkyletherphosphate können 1-10 Ethylenoxid- oder Propylenoxid- oder eine Kombination aus Ethylenoxid- und Propylenoxideinheiten enthalten.

### Amphotere Tenside:

Exemplarisch jedoch nicht limitierend sind folgende amphotere Tenside als Bestandteil der Formulierungen geeignet:
Alkylbetaine, Alkylamidobetaine, Sulfobetaine, Acetate und Diacetate, Imidazoline, Propionate und Alkylaminoxide.
Die Alkyl- und Acylgruppen enthalten hierbei 8-19 C-Atome.

### Nichtionische Tenside:

Exemplarisch jedoch nicht limitierend sind folgende nichtionische Tenside als Bestandteil der Formulierungen geeignet:
Alkylethoxylate, Arylethoxylate, ethoxylierte Ester, Polyglykolamide, Polysorbate, Glycerinfettsäure-Ethoxylate, Alkylphenolpolyglykolether und Zuckertenside wie z.B. Alkylglycoside.

### Kationische Tenside:

Bei kationischen Tensiden wird unterschieden zwischen reinen kationischen Tensiden und kationischen Polymeren.

### Reine kationische Tenside:

Exemplarisch jedoch nicht limitierend sind folgende nichtionische Tenside als Bestandteil der Formulierungen geeignet:
Monoalkylquats, Dialkylquats, Trialkylquats, Tetraalkylquats, Benzylam-moniumsalze, Pyridinsalze, Alkanolammoniumsalze, Imidazolinsalze, Oxazolinsalze, Thiazolinsalze, Salze von Aminoxiden, Sulfonsalze, wobei der Begriff "quat" das Vorliegen mindestens einer quaternären Ammoniumgruppe impliziert.

### Kationische Polymere:

Insbesondere für "2-in-1"-Shampoos werden neben den reinen kationischen Tensiden auch kationisch modifizierte Polymere eingesetzt. Eine umfassende Beschreibung dieser Polymere liefern US 5,977,038 und WO 01 /41720 A1. Bevorzugt sind hierbei kationische Polyacrylamide, kationische Eiweiß-derivate, Hydroxyalkylcelluloseether und kationische Guar Derivate. Besonders bevorzugt sind kationische Guar Derivate mit dem CTFA Namen Guar Hydroxypropyltrimonium Chloride. Diese Typen sind erhältlich unter den Handelsnamen Cosmedia Guar C 261 (Henkel), Diagum P 5070 (Diamalt), Jaguar C-Typen und Jaguar EXCEL von Rhodia.

### Hilfsstoffe:

Hilfsstoffe als Inhaltstoffe insbesondere von kosmetischen Formulierungen werden beschrieben in: A. Domsch, Die kosmetischen Präparate, Verlag für chem. Industrie, 4. Auflage, 1992; und in: Kosmetikjahrbuch 1995, Verlag für chemische Industrie, 1995. Exemplarisch jedoch nicht limitierend sind folgende Hilfsstoffe als Bestandteil der Formulierungen geeignet:
Anorganische und organische Säuren, Basen und Puffer, Salze, Alkohole wie z.B. Ethanol, Isopropanol, Ethylenglykol, Polyethylenglykol, Propylenglykol, Polypropylenglykol, Glykolether und Glycerin, Verdicker, Stabilisatoren für Emulsionen wie z.B. Xanthan Gum, Rückfetter, Konservierungsmittel, Schaumstabilisatoren, Entschäumer, Perlglanz und Trübungsmittel wie z.B. Glykoldistearate und Titandioxid, Kollagenhydrolysat, Keratinhydrolysat, Seidenhydrolysat, Anti-schuppenwirkstoffe wie z.B. Zinkpyrithion, Salicylsäure, Selendisulfid, Schwefel und Teerpräparate, polymere Emulgatoren, Vitamine, Farbstoffe, Stoffe zur Filterung von UV-Strahlen, Bentonite, Parfumöle, Duftstoffe, Styling Polymere, Feuchtigkeitsspender, Pflanzenextrakte und weitere natürliche oder naturidentische Rohstoffe.

Es ist bekannt, dass durch den Zusatz von öl- und wasserlöslichen UV-Filtern oder Kombinationen von UV-Filtern in kosmetischen Formulierungen zur Pflege und Behandlung von keratinhaltigen Substraten wie menschliches und tierisches Haar der Abbau von Farbstoffen und somit das Ausbleichen und Verblaßen von gefärbten keratinhaltigen Substraten durch UV-Strahlung entscheidend vermindert oder sogar gänzlich verhindert werden kann.

### Inhaltsstoffe für Haarfärbemittel:

Farbstoffe und andere Inhaltsstoffe von Haarfärbemitteln werden beschrieben in: A. Domsch, Die kosmetischen Präparate, Verlag für chem. Industrie, 4. Auflage, 1992. Farbstoffe werden beschrieben in: Verordnung über Kosmetische Mittel (Kosmetik Verordnung), Bundesgesetzblatt 1997, Teil I S. 2412, § 3 und Anlage 3 und in European Community (EC) Directive, 76/68/EEC, Annex IV.

Im Folgenden werden Haarfärbemittel unterschieden in permanente, semipermanente und temporäre Haarfärbemittel.

### Permanente Haarfärbemittel:

Dauerhafte Färbungen die auch durch mehrere Haarwäschen (mehr als 10) nicht ausgewaschen werden entstehen durch chemische Reaktion zwischen Farbstoffvorprodukten unter oxidative Bedingungen durch Wasserstoffperoxid. Hierbei bestimmt die Mischung der entsprechenden Komponenten das erzielbare Farbergebnis.

Man unterscheidet bei den Vorprodukten zwischen Oxidationsbasen (Entwickler) und Kupplungskomponenten (Modifizierer).

### Oxidationsbasen:

Exemplarisch jedoch nicht limitierend sind folgende Oxidationsbasen als Bestandteil der Formulierungen geeignet:
m- und p-Phenylendiamine (Diaminobenzole), deren N-substituierte Derivate und Salze, N-substituierte Derivate des o-Phenylendiamins, o-, m- und p-Toluylendiamine (Methyldiaminobenzole), deren N-substituierte Derivate und Salze, p-Amino-diphenylamin, - hydrochlorid und -sulfat, o-, m- und p-Aminophenol und -hydrochlorid, 2,4-Diaminoisosulfat (4-Methoxy-m-phenylen-diaminsulfat), o-Chlor-p-phenylendiaminsulfat, Pikra-minsäure (2,4-Dinitro-6-aminophenol) und 2,4-Dinitro-1-naphtol-sulfonsäure sowie deren Natriumsalz.

### Kupplungskomponenten:

Exemplarisch jedoch nicht limitierend sind folgende Kupplungskomponenten als Bestandteil der Formulierungen geeignet:
Hydrochinon (1,4-Dihydroxybenzol), Resorcin (1,3-Dihydroxybenzol), Brenzcatechin (1,2-Dihydroxybenzol), α-Naphtol (1-Hydroxynaphtalin), Pyrogallol (1,2,3-Trihydroxybenzol) und 2,6-Diaminopyridin.

Üblicherweise werden Oxidationsbasen und Kupplungskomponenten mit Tensiden in Ölin-Wasser Emulsionen eingearbeitet, jedoch sind auch einfache Lösungen oder Shampoos als Formulierungen bekannt. Die Formulierungen enthalten darüber hinaus Antioxidantien wie z.B. Natriumsulfit, Natriumdithionit, Ascorbinsäure oder Thioglykolsäure zur Stabilisierung der Vorstufen und werden mit alkalischen Substanzen wie z.B. Ammoniak auf einen pH-Wert zwischen 8 und 12 (bevorzugt 9-11) eingestellt. Außerdem werden Tenside als Netzmittel, Komplexbildner für Schwermetalle, Duftstoffe zum Überdecken des Ammoniakgeruchs, Conditioner zum Verbessern des Haargefühls und zum Schutz des Haares und Lösungsmittel wie Ethanol, Ethylenglykol, Glycerin oder Benzylalkohol zugesetzt.

Typischerweise werden permanente Haarfärbemittel als 2-Komponenten-Syteme angeboten bestehend aus Farblösung, -Creme oder -Shampoo die oben beschrieben ist und aus der Entwicklerlösung. Die Entwicklerlösung enthält hierbei zwischen 6-12 Gew.% Wasserstoffperoxid und kann optional auch mit Bestandteilen der die Farbkomponenten enthaltenden Formulierung versetzt werden. Die Peroxidlösung muß hierbei jedoch sorgfältig stabilisiert sein.

### Semipermanente Haarfärbemittel:

Semipermanente Färbungen wurden entwickelt, um die Färbung für 6-10-malige Wäsche mit Shampoo aufrecht zu erhalten. Hierbei werden sogenannte direktziehende Farbstoffe verwendet, die im Wesentlichen der Gruppe der Nitro-, Azo- und Anthrachinonfarbstoffe gehören. Diese Farbstoffmoleküle sind klein genug, um in das Haar zu penetrieren. Typischerweise eingesetzte Formulierungen sind Lösungen, Cremes, Shampoos oder auch Aerosolschäume. Die Zusammensetzung ist vergleichbar mit den die Farbkomponente enthaltenden Formulierungen die als permanente Haarfärbungen werden.

### Temporäre Haarfärbemittel:

Temporäre Färbungen, auch Tönungen genannt, enthalten im Unterschied zu den semipermanenten Haarfärbemitteln größere Farbstoffmoleküle, die nicht in der Lage sind in das Haar einzudringen. Sie wurden entwickelt, um die Färbung für 1-6 Wäschen aufrecht zu erhalten. Typischerweise werden hier Azo- und basische Farbstoffe sowie, Azin- und Thiazinderivate eingesetzt. Für die Zusammensetzung der Formulierungen gilt das bei den semipermanenten und permanenten Haarfärbemitteln gesagte. Farbstoffe und andere Inhaltsstoffe von Haarfärbemitteln werden beschrieben in: A. Domsch, Die kosmetischen Präparate, Verlag für chem. Industrie, 4. Auflage, 1992. Farbstoffe werden beschrieben in: Verordnung über Kosmetische Mittel (Kosmetik Verordnung), Bundesgesetzblatt 1997, Teil I S. 2412, § 3 und Anlage 3 und in European Community (EC) Directive, 76/68/EEC, Annex IV.

Als besonders vorteilhaft für die Verwendung von die erfindungsgemäßen Polyharnstoff- und Polyurethan-Polyorganosiloxan-Verbindungen enthaltenden Mischungen in kosmetischen Formulierungen wurden die folgenden, die Erfindung jedoch nicht limitierenden Rezepturen gefunden, in denen jeder funktionelle Wirkstoff als einzelne Verbindung oder als Gemisch mehrerer Verbindungen dieser Kategorie auftreten kann.

Eine typische, erfindungsgemäße, die Erfindung jedoch nicht limitierende Shampoo-Formulierung zur Pflege und Konditionierung von Haaren besteht aus folgenden Bestandteilen in Gew.-%, jeweils bezogen auf die Gesamtformulierung:

| | |
|---|---|
| 0,01 - 10% | erfindungsgemäße Polyharnstoff- und/oder Polyurethan-Polyorganosiloxan-Verbindungen |
| 2 - 15% | Anionisches Tensid |
| 0 - 10% | Amphoteres Tensid |
| 0 - 15% | Nichtionisches Tensid |
| 0 - 10% | Kationisches Tensid |
| 0 - 10% | Silikone Konditioniermittel (Co-Adjuvantien) |
| 0 - 10% | Hilfsstoffe |
| auf 100 % | ergänzt durch Wasser. |

Eine spezielle, die Erfindung jedoch nicht limitierende Shampoo-Formulierung besteht aus folgenden Bestandteilen in Gew.%:

| | |
|---|---|
| 0,1 - 12 % | erfindungsgemäße Polyharnstoff- und/oder |
| | Polyurethan-Polyorganosiloxan-Verbindungen |
| 1 - 35 % | Natrium oder Ammonium Lauryl- bzw. |
| | Laurethsulfat (20 - 30%) |
| 1 - 6 % | Cocoamidopropylbetain (25 - 35%) |
| 0 - 3 % | Guar Hydroxypropyltrimonium Chlorid |
| 0 - 5 % | Polyquaternium-10 |
| 0 - 12 % | Silikone Konditioniermittel (Co-Adjuvantien) |
| 0,01 - 1% | Dinatrium EDTA |
| 0,01 - 1% | Phenoxyethynol (und) Methylparaben (und) |
| | Butylparaben (und) Ethylparaben (und) |
| | Propylparaben |
| 0 - 1 % | Parfum (Duftstoff) |
| 0 - 1 % | Farbstoffe |
| 0 - 1 % | Zitronensäure |
| 0 - 2 % | Natriumchlorid |
| auf 100 % | ergänzt durch Wasser. |

Eine typische, erfindungsgemäße, die Erfindung jedoch nicht limitierende Haar-spülung zur Pflege und Konditionierung von Haaren besteht aus folgenden Bestandteilen in Gew.-%:

| | |
|---|---|
| 0,1 - 15% | erfindungsgemäße Polyharnstoff- und/oder Polyurethan-Polyorganosiloxan-Verbindungen |
| 0 - 10 % | Amphoteres Tensid |
| 0,1 - 15% | Nichtionisches Tensid |
| 0 - 10 % | Kationisches Tensid |
| 0 - 15 % | Silikone Konditioniermittel (Co-Adjuvantien) |
| 0 - 20 % | Hilfsstoffe |
| auf 100 % | ergänzt durch Wasser. |

Eine spezielle, die Erfindung jedoch nicht limitierende Zusammensetzung einer Haarspülung besteht aus folgenden Bestandteilen in Gew.-%:

| | |
|---|---|
| 0,5 - 15% | erfindungsgemäße Polyharnstoff- und/oder Polyurethan-Polyorganosiloxan-Verbindungen (als 43,5%-ige Emulsion in Wasser mit nichtionischen Emulgatoren) |
| 0 - 15 % | Silikone Konditioniermittel (Co-Adjuvantien) |
| 0 - 10 % | Cetrimonium Chlorid (25 - 35 %) |
| 0 - 3 % | Guar Hydroxypropyltrimonium Chlorid |
| 1 - 10 % | Cetearyl Alkohol |
| 0 - 10 % | Glycerin |
| 0,01 - 1% | Phenoxyethynol (und) Methylparaben (und) Butylparaben (und) Ethylparaben (und) Propylparaben |
| 0 - 1 % | Parfum (Duftstoff) |
| 0 - 1 % | Farbstoffe |
| 0 - 1 % | Zitronensäure |
| auf 100 % | ergänzt durch Wasser. |

Eine typische, erfindungsgemäße Haarpflege-Kur zur Pflege und Konditionierung von Haaren besteht aus folgenden Bestandteilen in Gew.-%:

| | |
|---|---|
| 0,4 - 20% | erfindungsgemäße Polyharnstoff- und/oder Polyurethan-Polyorganosiloxan-Verbindungen |
| 0 - 15 % | Nichtionisches Tensid |
| 0 - 10 % | Kationisches Tensid |
| 0 - 20 % | Silikone Konditioniermittel (Co-Adjuvantien) |
| 0 - 20 % | Hilfsstoffe |
| auf 100 | ergänzt durch Wasser. |

Eine spezielle Haarpflege-Kur, besteht aus folgenden Bestandteilen in Gew.-%:

| | |
|---|---|
| 1 - 20 % | erfindungsgemäße Polyharnstoff- und/oder Polyurethan-Polyorganosiloxan-Verbindungen (als 43,5%-ige Emulsion in Wasser mit nichtionischen Emulgatoren) |
| 0,5 - 10% | Stearyl Alcohol (und) Steareth-7 (und) Steareth-10 |
| 0 - 20 % | Silikone Konditioniermittel (Co-Adjuvantien) |
| 0 - 10% | Cetrimonium Chlorid (25 - 35 %) |
| 0 - 3 % | Guar Hydroxypropyltrimonium Chlorid |
| 0 - 5 % | Dimethicone |
| 0 - 5 % | Paraffinöl |
| 1 - 10 % | Stearyl Alkohol |
| 0 - 10 % | Glycerin |
| 0,01 - 1% | Phenoxyethynol (und) Methylparaben (und) Butylparaben (und) Ethylparaben (und) Propylparaben |
| 0 - 1 % | Parfum (Duftstoff) |
| 0 - 1 % | Farbstoffe |
| 0 - 1 % | Zitronensäure |
| 0 - 2 % | Natriumchlorid |
| auf 100 % | ergänzt durch Wasser. |

Eine ganz spezielle Haarpflege-Kur, besteht aus folgenden Bestandteilen in Gew.-%:

| | |
|---|---|
| 2 - 5 % | erfindungsgemäße Polyharnstoff- und/oder Polyurethan-Polyorganosiloxan-Verbindungen (als 43,5%-ige Emulsion in Wasser mit nichtionischen Emulgatoren) |
| 0 - 5 % | Silikon-Konditioniermittel (Co-Adjuvantien) |
| 0 - 2 % | Cetrimonium Chlorid (25 - 35%) |
| 0,5 - 5 | Glycerin |
| 0,25 - 2,5% | Propylenglykol |
| 0,05 - 0,2% | Parfum |
| 0,1 - 0,5 | Polysorbat 20 |
| auf 100 % | ergänzt durch Wasser. |

Eine typische, erfindungsgemäße, die Erfindung jedoch nicht limitierende die Farbstoffe enthaltende Formulierung zur temporären, semi-permanenten oder permanenten Haarfärbung Pflege und Konditionierung der Haare besteht aus folgenden Bestandteilen in Gew.-%:

| | |
|---|---|
| 0,1 - 10% | erfindungsgemäße Polyharnstoff- und/oder Polyurethan-Polyorganosiloxan-Verbindungen |
| 1 - 10% | Haarfarbstoffvorstufen oder Farbstoffe je nach gewünschter Haarfarbe |
| 0 - 15% | Anionisches Tensid |
| 0 - 10% | Amphoteres Tensid |
| 0 - 10% | Nichtionisches Tensid |
| 0 - 10% | Kationisches Tensid |
| 0 - 1% | Natriumsulfit |
| 0 - 5% | Puffer |
| 0 - 10% | Silikone Konditioniermittel (Co-Adjuvantien) |
| 0 - 10% | Hilfsstoffe |
| auf 100 % | Wasser. |

Eine spezielle Farbcreme für die permanente Haarfärbung besteht aus folgenden Bestandteilen in Gew.-%:

| | |
|---|---|
| 0,1 - 10% | erfindungsgemäße Polyharnstoff- und/oder Polyurethan-Polyorganosiloxan-Verbindungen (als 20%-ige Emulsion in Wasser mit nichtionischen Emulgatoren) |
| 1 - 5% | Haarfarbvorstufen oder Farbstoffe je nach gewünschter Haarfarbe |
| 2 - 15% | Anionisches Tensid |
| 0 - 10% | Amphoteres Tensid |
| 0 - 10% | Nichtionisches Tensid |
| 0 - 10% | Kationisches Tensid |
| 0,1 - 1% | Natriumsulfit |
| 0,1 - 5% | Puffer für pH = 8 - 12 |
| 0 - 10 % | Silikone Konditioniermittel (Co-Adjuvantien) |
| 0 - 10% | Hilfsstoffe |
| auf 100 % | Wasser. |

Eine spezielle, erfindungsgemäße, die Erfindung jedoch nicht limitierende Farblösung für die permanente Haarfärbung besteht aus folgenden Bestandteilen in Gew.-%:

| | |
|---|---|
| 0,1 - 10% | erfindungsgemäße Polyharnstoff- und/oder Polyurethan-Polyorganosiloxan-Verbindungen (als 20 %-ige Emulsion in Wasser mit nichtionischen Emulgatoren) |
| 1 - 5 % | Haarfarbvorstufen oder Farbstoffe je nachgewünschter Haarfarbe |
| 0,1 - 1% | Natriumsulfit |
| 5 - 15 % | Propylenglykol |
| 5 - 15 % | Ammoniak (28 %) |
| 10 - 30% | Ölsäure |
| 5 - 15 % | Isopropanol |
| 10 - 30 % | Alkanolamid |
| 0 - 10 % | Silikone Konditioniermittel (Co-Adjuvantien) |
| auf 100 % | Wasser. |

Eine typische, erfindungsgemäße, die Erfindung jedoch nicht limitierende Entwicklerformulierung für die permanente Haarfärbung besteht aus folgenden Bestandteilen in Gew.-%:

| | |
|---|---|
| 0,1 - 10% | erfindungsgemäße Polyharnstoff- und/oder Polyure-than-Polyorganosiloxan-Verbindungen |
| 10 - 30% | Wasserstoffperoxid (30%) |
| 0 - 15% | Anionisches Tensid |
| 0 - 10% | Amphoteres Tensid |
| 0 - 10% | Nichtionisches Tensid |
| 0 - 10% | Kationisches Tensid |
| 0 - 5 % | Puffer bzw. Säure für pH = 2-6 |
| 0 - 10 % | Silikone-Konditioniermittel (Co-Adjuvantien) |
| 0 - 10% | Hilfsstoffe |
| auf 100 % | Wasser |

Eine spezielle, erfindungsgemäße, die Erfindung jedoch nicht limitierende Entwickler Creme für eine permanente Haarfärbung besteht aus folgenden Bestandteilen in Gew.-%:

| | |
|---|---|
| 0,1 - 5% | erfindungsgemäße Polyharnstoff- und/oder Polyurethan-Polyorganosiloxan-Verbindungen (als 20%-ige Emulsion in Wasser mit nichtionischen Emulgatoren) |
| 10 - 30% | Wasserstoffperoxid (30%) |
| 0 - 5 % | Silikon-Konditioniermittel (Co-Adjuvantien) |
| 1 - 10% | Cetearyl Alkohol |
| 0,5 - 5% | Trideceth-2 Carboxamid MEA |
| 0,5 - 5% | Ceteareth-30 |
| 0,5 - 5% | Glycerin |
| 0,05 - 2% | Pentasodium Pentetate (Pentanatrium Diethylentriamin-pentaacetat |
| 0,05 - 2% | Natriumstannat |
| 0,05 - 2% | Tetranatriumpyrophosphat |
| auf 100 % | Wasser. |

Es wurde hierbei erkannt, dass sich die erfindungsmäßen Lösungen oder Mischungen vorzugsweise zur Herstellung von kosmetischen Formulierungen eignen, wie für die Behandlung, Konditionierung, Reinigung und/oder Pflege gefärbter oder zu färbender Substrate.

D.h. die mindestens eine erfindungsgemäße Polyharnstoff- und/oder Polyurethan-Polyorganosiloxan-Verbindung enthaltenden Formulierungen können insbesondere für die Reinigung, Pflege und Konditionierung von faserartigen oder flächigen Substraten eingesetzt werden und wenn diese gefärbt sind und deren Farbeindruck weitestgehend erhalten bleiben soll.

Die mindestens eine erfindungsgemäße Polyharnstoff- und/oder Polyurethan-Polyorganosiloxan-Verbindung enthaltenden Formulierungen können weiterhin für die Reinigung, Pflege und die Behandlung und die Konditionierung keratinhaltiger Substrate dienen, da sie als Reinigungsmittel für Wolle, zur Wäsche und/oder Erhöhung des Volumens und/oder der Kämmbarkeit und/oder des Glanzes und/oder zur Verminderung des Auswaschens der Farbe von und aus gefärbten oder von gleichzeitig zu färbenden keratinhaltigen Substraten wie z.B. menschlichem und tierischem Haar geeignet sind.

Weiterhin können die mindestens eine erfindungsgemäße Polyharnstoff- und/oder Polyurethan-Polyorganosiloxan-Verbindung enthaltenden Formulierungen insbesondere für die Reinigung, die Behandlung und Pflege, Reinigung und Pflege keratinhaltiger Fasern bzw. Haare vor, während und/oder nach dem Färbevorgang verwendet werden, da die hiermit formulierten Haarfärbemittel gleichzeitig zur Verbesserung der Weichheit und/oder zur Verminderung der Nass- und Trockenkämmkräfte und/oder zur Erhöhung des Glanzes und/oder zur Erhöhung des Haar-volumens und/oder zur Verminderung des Auswaschens von Farbstoffen von und aus getönten und gefärbten Haaren führen.

### Weichspülerformulierungen

Hinsichtlich der Darrreichungsform ist es einerseits möglich, die erfindungsgemäßen Polyharnstoff- und/oder Polyurethan-Polyorganosiloxan-Verbindungen in nicht transparente Weichspülerdispersionen oder Weichspüleremulsionen bzw. transparente Mikroemulsionen oder Lösungen einzuarbeiten.

Typische weitere Komponenten für derartige nicht transparente oder transparente Formulierungen sind:
- quaternäre Ammoniumverbindungen, bevorzugt Alkansäureester-einheiten
   enthaltende quaternäre Ammoniumverbindungen als Weichmacher,
- organische Lösungsmittel, bevorzugt ein und mehrwertige Alkohole, wie Ethanol, 2-Propanol, Ethylenglykol, 1.2-Propylenglykol, Hexylenglykol, Dipropylenglykol, Ester und Ether von Glykolen und Oligo-glykolen, wie Dipropylenglykolmonobutylether, Tripropylen-monomethylether, Diethylenglykoldiacetat, zur Verbesserung der Löslichkeit und Transparenz der Formulierung,
- Diole und höhere Alkohole längerkettiger Kohlenwasserstoffe, beispielsweise 2,2,4-Trimethyl-1,3-Pentandiol, zur Steigerung der Solubilisierbarkeit der Weichmacherkomponenten,
- nichtionogene Tenside, bevorzugt Alkoxylate von verzweigten bzw. unverzweigten C₈ bis C₄₀-Alkoholen und Fettsäureester von Alkylenoxiden zur Emulsionsstabilisierung bzw. Mikroemulsionsherstellung
- Parfüms
- Viskositätsregler
- Farbstoffe
- Konservierungsmittel.

Die aufgeführten zusätzlichen Funktionalkomponenten und bevorzugte Vertreter sind beispielsweise aus US 6,376,455 bekannt.

Weiterhin ist es möglich, die erfindungsgemäßen Polyharnstoff- und/oder Polyurethan-Polyorganosiloxan-Verbindung im Kontext von Wäscheauffrischer-Systemen auf feste Träger aufzubringen und diese dann im Wäsche-trockner in Kontakt mit aufzufrischenden und/oder weich zu machenden Textilien zu bringen. Geträgerte WäscheauffrischerSysteme und deren Funktionalkomponenten sind beispielsweise aus US 4,824,582, US 4,808,086, US 4,756,850, US 4,749,596, US 3,686,025 bekannt.
Typische Komponenten für derartige geträgerte Wäscheauffrischersysteme sind:
- Fettamine bzw. deren Komplexe mit anionischen Tensiden als Konditioniermittel
- quaternäre Ammoniumverbindungen, bevorzugt Alkansäureester-einheiten enthaltende quaternäre Ammoniumverbindungen als Weichmacher
- nichtionogene Weichmacher, beispielsweise auf Basis von Sorbitanestern, Fettalkohlalkoxylaten
- "soil release agents", beispielsweise auf Basis von Celluloseethern, Guar Gum, Terephthalsäureblockcopolymeren.

Das Trägermaterial ist ein schwammartiges oder poröses blattartiges Material, welches eine hinreichende Kapazität zur Aufnahme der Wäscheauffrischer-Formulierung aufweist. Es kommen "woven"- und "nonwoven"- Materialien zum Einsatz. Es handelt sich um Materialien auf Basis natürlicher oder synthetischer Polymerer, wie Wolle, Baumwolle, Sisal, Leinen, Cellulose-estern, Polyvinylverbindungen, Polyolefinen, Polyamiden, Polyurethanen, Polyestern.

Die vorliegende Erfindung wird durch die folgenden Beispiele weiter veranschaulicht.

### BEISPIELE

### Beispiel 1

In einem Kolben werden bei Raumtemperatur unter N₂-Atmosphäre 58 g Methoxypropylacetat, 7,19 g (10,8 mmol) eines isocyanat-terminierten Polyalkylenoxid-Derivates der Struktur 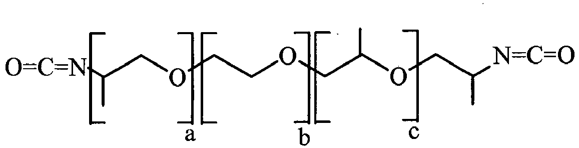 mit a+c=3.6 und b= 9
und 50 g eines Aminosiloxans der Struktur 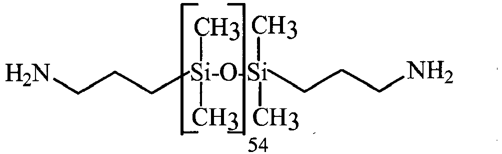 vermischt. Die Temperatur steigt auf 30 °C an. Die Mischung wird für 10 Stunden auf 95 bis 100°C erhitzt.

Im Ergebnis der Reaktion wird eine klare gelbe Lösung gewonnen, welche ein Polymer mit folgendem Strukturelement enthält: 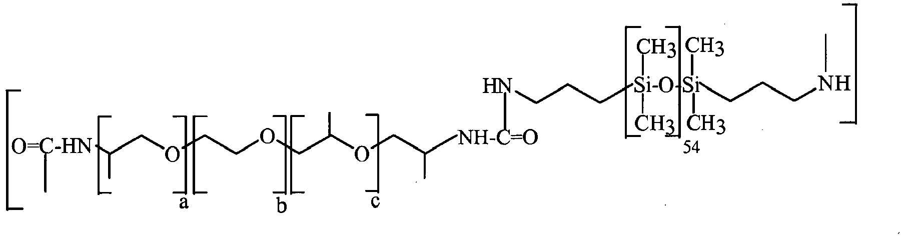 mit a+c=3.6 und b= 9

### Beispiel 2

In einem Kolben werden bei Raumtemperatur unter N₂ Atmosphäre 58 g Methoxypropylacetat, 5,34 g (8,04 mmol) eines isocyanat-terminierten Polyalkylenoxidderivats der Struktur 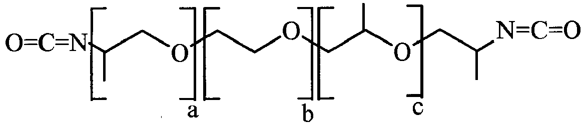 mit a+c = 3.6 und b = 9 und
1,69 g (16,1 mmol) H₂NCH₂CH₂CH₂N(CH₃)₂ vorgelegt und unter Rührung für 6 Stunden auf 95-100 °C erhitzt.
Anschließend wird eine Mischung, bestehend aus 50 g (8,04 mmol) eines Epoxysiloxans der Struktur

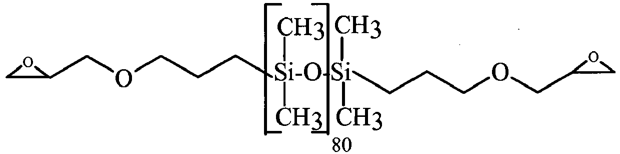

1g deionisierten Wassers und 0,97 g (16,1 mmol) Essigsäure, zugegeben. Die Gesamtmischung wird für 10 Stunden auf 95-100 °C erhitzt.

Im Ergebnis der Reaktion wird eine rötliche, trübe Lösung gewonnen, die ein Polymer mit folgendem Strukturelement aufweist: 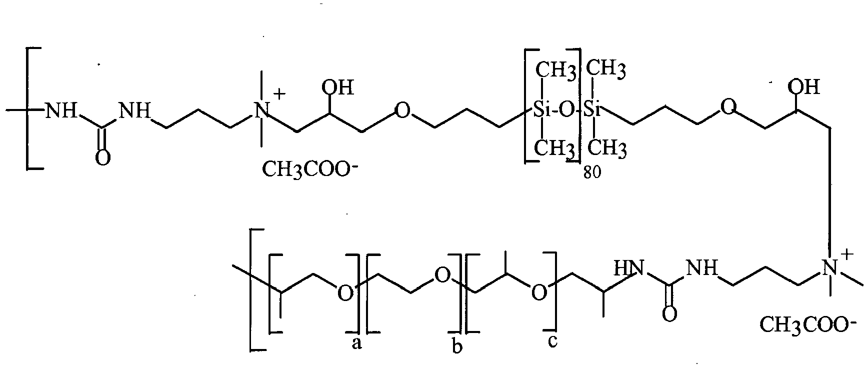 mit a+c=3.6 und b= 9

Die Polymeren gemäß Beispielen 1 und 2 können in einer Menge von beispielsweise 0,5-3 % in pulverförmige und flüssige Waschmittel auf Basis anionischer und/oder nichtionogener Tenside eingearbeitet werden, und üben dort insbesondere ihre weichmachende Wirkung auf die zu reinigenden Fasermaterialien bei hoher Substantivität aus.

## Patentansprüche

1. Polyharnstoff und/oder Polyurethan-Polyorganosiloxan-Verbindungen a), enthaltend mindestens ein Strukturelement der Formel (1): 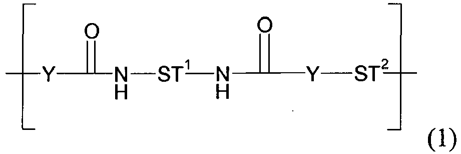 worin
ST¹ und ST² unabhängig voneinander ausgewählt werden aus zwei- oder höherwertigen, geradkettigen, cyclischen oder verzweigten, gesättigten, ungesättigten oder aromatischen, substituierten oder unsubstituierten Kohlenwasserstoffresten mit bis zu 1000 Kohlenstoffatomen (wobei die Kohlenstoffatome einer gegebenenfalls enthaltenen Polyorganosiloxaneinheit nicht mitgezählt werden), die eine oder mehrere Gruppen, ausgewählt aus
-O-, -NH-, -_{NR}³-, 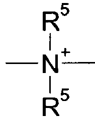 und einer Polydiorganosiloxaneinheit mit 2 bis 1000 Siliciumatomen enthalten können, worin
R³ ein geradkettiger, cyclischer oder verzweigter, gesättigter, ungesättigter oder aromatischer Kohlenwasserstoffrest mit bis zu 40 Kohlenstoffatomen ist, der eine oder mehrere Gruppen, ausgewählt aus -O-, -C(O)- und -NH- enthalten kann, und,
R⁵ ein geradkettiger, cyclischer oder verzweigter, gesättigter, ungesättigter oder aromatischer Kohlenwasserstoffrest mit bis zu 100 Kohlenstoffatomen ist, der eine oder mehrere Gruppen, ausgewählt aus -O-, -C(O) - und -NH- enthalten kann, oder R⁵ ein zweiwertiger Rest ist, der cyclische Strukturen innerhalb der Reste ST¹ und/oder ST² ausbildet,
und wobei wenn eine Mehrzahl von Resten ST¹ vorliegt, diese gleich oder verschieden voneinander sein können, und wenn eine Mehrzahl von Resten ST² vorliegt, diese gleich oder verschieden voneinander sein können,
mit der Maßgabe, dass
mindestens einer der Reste ST¹ einen Polyorganosiloxanrest umfasst und mindestens einer der Reste ST² einen Polyalkylenoxidrest umfasst, oder
mindestens einer der Reste ST² einen Polyorganosiloxanrest umfasst und mindestens einer der Reste ST¹ einen Polyalkylenoxidrest umfasst.
oder
Polyharnstoff- und/oder Polyurethan-Polyorganosiloxan-Verbindungen b), enthaltend mindestens ein Strukturelement der Formel (3): 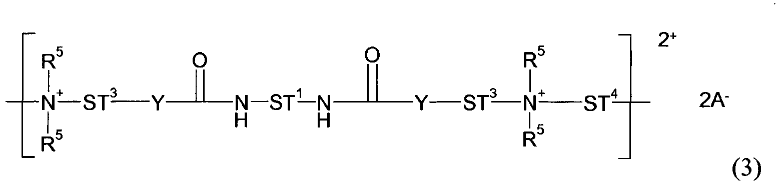 worin
Sitz ausgewählt wird aus zwei- oder höherwertigen, geradkettigen, cyclischen oder verzweigten, gesättigten, ungesättigten oder aromatischen, substituierten oder unsubstituierten Kohlenwasserstoffresten mit bis zu 1000 Kohlenstoffatomen (wobei die Kohlenstoffatome einer gegebenenfalls enthaltenen Polyorganosiloxaneinheit nicht mitgezählt werden), die eine oder mehrere Gruppen, ausgewählt aus -O-, -NH-, NR³-, 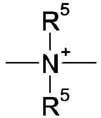 und einer Polydiorganosiloxaneinheit mit 2 bis 1000 Siliciumatomen enthalten können,
worin R³ und R⁵ wie oben definiert sind, und
ST³ ein geradkettiger oder cyclischer oder verzweigter, gesättigter oder ungesättigter oder aromatischer, substituierter oder unsubstituierter Kohlenwasserstoffrest mit 2 bis 100 Kohlenstoffatomen ist, der durch
-O-,-NH-, NR³- substituiert sein kann, worin R³ wie oben definiert ist, und
ST⁴ ein geradkettiger oder cyclischer oder verzweigter, gesättigter oder ungesättigter oder aromatischer, substituierter oder unsubstituierter Kohlenwasserstoffrest mit 2 bis 100 Kohlenstoffatomen (wobei die Kohlenstoffatome einer gegebenenfalls enthaltenen Polyorganosiloxaneinheit nicht mitgezählt werden), der durch -O-,-NH-, -NR³- und durch eine Polydiorganosiloxaneinheit mit 2 bis 200 Siliciumatomen substituiert sein kann, worin R³ wie oben definiert ist, und
A⁻ ein organisches oder anorganisches Anion ist,
mit der Maßgabe, dass mindestens einer der Reste ST¹, ST⁴ einen Polydiorganosiloxan-Rest enthält,
und worin die Polyharnstoff und/oder Polyurethan-Polyorganosiloxan-Verbindungen b) erhalten werden durch die Umsetzung einer Verbindung der Formel 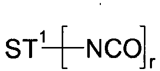 worin ST¹ wie oben definiert ist, mit einer Verbindung der Formel
HY-ST³-NR⁵₂,
worin Y, ST³ und R⁵ wie oben definiert sind,
und anschließend mit einer Verbindung der Formel
Q-ST^{4V}-Q,
umfasst, worin Q ein zur Alkylierung einer Aminogruppe befähigter Rest ist, und ST^{4V} gemeinsam mit dem aus Q nach der Quaternierungsreaktion hervorgehenden Molekülteil den Rest ST⁴ bildet,
Y -NR²- oder -O- ist, worin
R² Wasserstoff oder ein geradkettiger, cyclischer oder verzweigter, gesättigter, ungesättigter oder aromatischer Kohlenwasserstoffrest mit bis zu 40 Kohlenstoffatomen ist, der eine oder mehrere Gruppen, ausgewählt aus -O-, -C(O)-,-NH- und -NR³- enthalten kann, worin R³ wie oben definiert ist,
oder Säureadditionsverbindungen und oder Salze davon.

2. Polyharnstoff- und/oder Polyurethan-Polyorganosiloxan-Verbindungen nach Anspruch 1, worin die Reste ST¹ ST², ST³ und ST⁴ zweiwertige Reste sind, so dass die Polyharnstoff- und/oder Polyurethan-Polyorganosiloxan-Verbindungen linear aufgebaut sind.

3. Polyhamstoff-Polyorganosiloxan-Verbindungen nach Anspruch 1 oder 2, worin Y ist: NH oder NR², worin R² wie oben definiert ist.

4. Polyharnstoff- und/oder Polyurethan-Polyorganosiloxan-Verbindungen nach einem oder mehreren der Ansprüche 1 bis 3, enthaltend mindestens einen Polyalkylenoxidrest.

5. Verfahren zur Herstellung von Polyharnstoff- und/oder Polyurethan-Polyorganosiloxan-Verbindungen nach einem oder mehreren der Ansprüche 1 bis 4, welches die Umsetzung einer Verbindung der Formel: 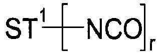 worin ST¹ wie oben definiert ist, und r ≥ 2 ist, mit einer Verbindung der Formel 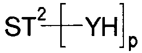 umfasst, worin ST² wie oben definiert ist, und p ≥ 2 ist, mit der Maßgabe, dass
die Polyharnstoff- und/oder Polyurethan-Polyorganosiloxan-Verbindungen mindestens einen Polyorganosiloxanrest und mindestens einen Polyalkylenoxidrest umfassen, und insbesondere der Rest ST¹ einen Polyorganosiloxanrest umfasst und der Rest ST² einen Polyalkylenoxidrest umfasst, oder
der Rest ST² einen Polyorganosiloxanrest umfasst und der Rest ST¹ einen Polyalkylenoxidrest umfasst.

6. Verfahren zur Herstellung von Polyharnstoff- und/oder Polyurethan-Polyorganosiloxan-Verbindungen nach Anspruch 5, worin die Verbindung der Formel 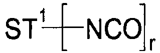 ausgewählt wird aus
aliphatischen, zwei- oder mehrwertigen, geradkettigen oder verzweigten Polyisocyanaten mit bis zu 15 Kohlenstoffatomen,
beispielsweise Hexamethylendiisocyanat, aliphatischen, zweioder mehrwertigen, cyclischen Polyisocyanaten mit bis zu 15 Kohlenstoffatomen, wie beispielsweise 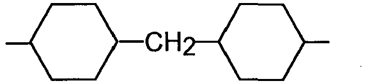 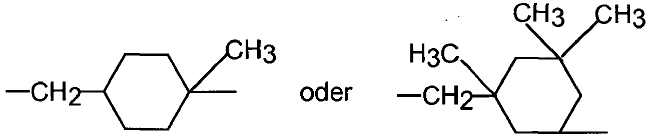 zwei- oder mehrwertige, aromatische Polyisocyanate mit bis zu 15 Kohlenstoffatomen,
beispielsweise auf Basis von 2,4-Toluyl, 2,6-Toluyl, Bis-phenylmethan- und Naphthylenstrukturen, wie 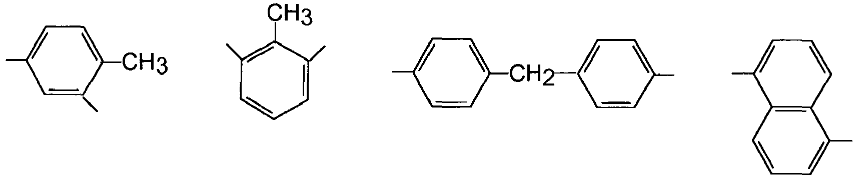 komplexeren Polyisocyanaten, wie
beispielsweise isocyanat-terminierte Polyether, bevorzugt ethylenoxid- und propylenoxidbasierte Polyether, beispielsweise hergestellt aus primären aminoterminierten Polyethern vom Typ Jeffamine^{®}, beispielsweise der ED- und T- . Serie (Huntsman Corp.),
isocyanat-terminierte Polyamide,
isocyanat-terminierte Polyharnstoffe,
isocyanat-terminierte Polyurethane, isocyanatoalkyl-funktionalisierte Polyorganosiloxane,
wie höherfunktionelle Polyisocyanate, die sich von primäre Aminogruppen tragenden Polyorganosiloxanen, mit kammartig und gegebenenfalls in α,ω-Position angeordneten primären Aminofunktionen ableiten.

7. Verfahren zur Herstellung von Polyharnstoff- und/oder Polyurethan-Polyorganosiloxan-Verbindungen nach einem der Ansprüche 5 oder 6, worin die Verbindung der Formel: 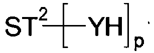 ausgewählt wird aus
aliphatischen, zweiwertigen oder mehrwertigen, geradkettige oder verzweigten Polyolen oder Polyaminen mit bis zu 15 Kohlenstoffatomen, beispielsweise Hexamethylendiamin oder -diol,
aliphatischen, zweiwertigen oder mehrwertigen, cyclischen Polyolen oder Polyaminen mit bis zu 15 Kohlenstoffatomen, beispielsweise auf Basis von Biscyclohexylmethan-Strukturen 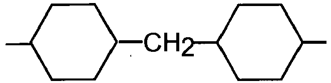 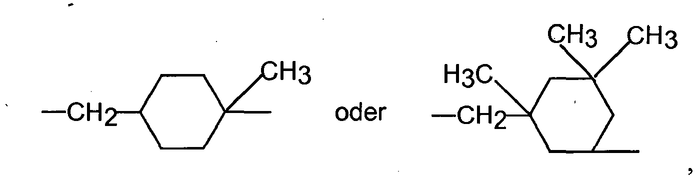 zwei- oder mehrwertigen, aromatischen Polyolen oder Polyaminen mit bis zu 15 Kohlenstoffatomen, beispielsweise auf Basis von 2,4-Toluyl, 2,6-Toluyl, Bis-phenylmethan- und Naphthylenstrukturen 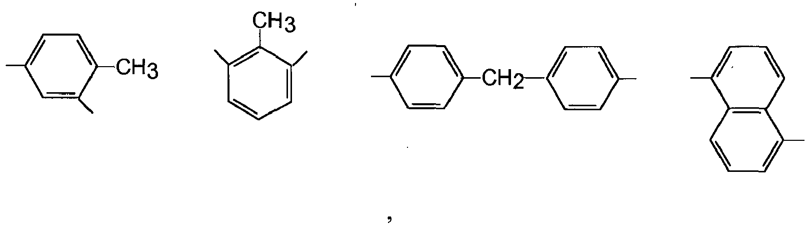 insbesondere Hexamethylendiamin, Phenylendiamine, Toluendiamine, Cyclohexandiamin, Ethylendiamin, Propylendiamin,
zwei- oder höherwertigen primären und/oder sekundären Amine oder Alkoholen, wie beispielsweise N(CH₂CH₂NH₂)₃, Diethylentriamin, Dipropylentriamin und höhere Oligomere, Glycerin und dessen alkoxylierte Derivate,
komplexere, zwei- oder höherwertigen Polyaminen oder Polyolen, wie primär und sekundär amino- oder hydroxylfunktionalisierte Präpolymere, welche ebenfalls höherfunktionalisiert sein können, wie beispielsweise
- primär oder sekundär amino- oder hydroxylterminierte Polyether, bevorzugt ethylenoxid- und propylenoxidbasierte Polyether, beispielsweise primär oder sekundär aminoterminierte Polyether vom Typ Jeffamine^{®}, beispielsweise der ED- und T-Serie (Huntsman Corp.)
- NH₂- terminierte Polyamide,
- NH₂- terminierte Polyharnstoffe,
- NH₂- terminierte Polyurethane,
- OH- terminierte Polyharnstoffe,
- OH- terminierte Polyurethane,
- OH- terminierte Polyester,
- α,ω-primär oder sekundär amino- oder hydroxyalkyl- oder hydroxypolyetheralkylfunktionalisierte Polyorganosiloxane, wie höherfunktionelle primäre oder sekundäre Amino- oder Hydroxyalkyl- oder Hydroxypolyetheralkylgruppen tragende Polyorganosiloxane die über kammartig und gegebenenfalls in α,ω Position angeordnete Amino- oder Hydroxylfunktionen verfügen.

8. Verfahren zur Herstellung von Polyharnstoff- und/oder Polyurethan-Polyorganosiloxan-Verbindungen nach einem oder mehreren der Ansprüche 1 bis 4, der Formel (3) 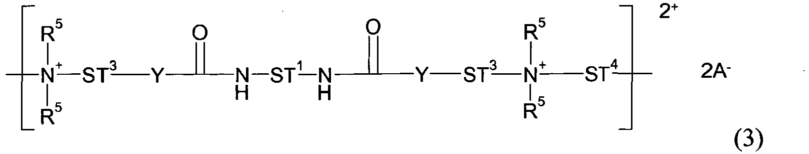 worin
A⁻, R⁵, ST¹, ST³, ST⁴ und Y wie oben definiert sind.
welches die Umsetzung einer Verbindung der Formel 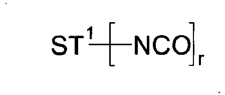 worin ST¹ wie oben definiert ist, mit einer Verbindung der Formel
HY-ST³-NR⁵₂,
worin Y, ST³ und R⁵ wie oben definiert sind,
und anschließend mit einer Verbindung der Formel
Q-ST^{4V}- Q,
umfasst, worin Q ein zur Alkylierung einer Aminogruppe befähigter Rest ist, und ST^{4V} gemeinsam mit dem aus Q nach der Quaternierungsreaktion hervorgehenden Molekülteil den Rest ST⁴ bildet.

9. Reaktives 1- oder 2-Komponenten-System, enthaltend mindestens eine Verbindung der Formel 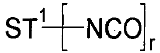 worin ST¹ wie oben definiert ist, und r ≥ 2 ist, worin die Isocyanatgruppen gegebenenfalls in an sich bekannter Weise blockiert sein können,
und mindestens eine Verbindung der Formel 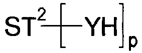 worin ST² wie oben definiert ist, und p ≥ 2 ist, mit der Maßgabe, dass
der Rest ST¹ einen Polyorganosiloxanrest umfasst und
der Rest ST² einen Polyalkylenoxidrest umfasst, oder
der Rest ST² einen Polyorganosiloxanrest umfasst und
der Rest ST¹ einen Polyalkylenoxidrest umfasst,
sowie gegebenenfalls Füllstoffe, Pigmente, weitere Bindemittel, Hilfsstoffe, Lösungsmittel.

10. Reaktives 1- oder 2-Komponenten-System, enthaltend mindestens ein Umsetzungsprodukt aus mindestens einer Verbindung der Formel 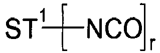 worin ST¹ wie oben definiert ist, und mindestens einer Verbindung der Formel
HY-ST³-NR⁵₂,
worin Y, ST³ und R⁵ wie oben definiert sind,
und mindestens eine Verbindung der Formel
Q-ST^{4V}-Q,
worin Q ein zur Alkylierung einer Aminogruppe befähigter Rest ist, und ST^{4V} gemeinsam mit dem aus Q nach der Quaternierungsreaktion hervorgehenden Molekülteil den Rest ST⁴ bildet,
sowie gegebenenfalls Füllstoffe, Pigmente, weitere Bindemittel, Hilfsstoffe, Lösungsmittel.

11. Verwendung der Polyharnstoff- und/oder Polyurethan-Polyorganosiloxan-Verbindungen nach einem oder mehreren der Ansprüche 1 bis 4, oder der reaktiven 1- oder 2-Komponentensysteme nach den Ansprüchen 9 oder 10 sowie den gehärteten Zusammensetzungen daraus zur Herstellung von Beschichtungen, Mitteln zur Oberflächenmodifizierung, Elastomeren, Duromeren, Klebstoffen, Primern für Metall- und Kunststoffoberflächen, Polymeradditiven, Waschmitteladditiven, rheologischen Mitteln, kosmetischen Mitteln, Fasermodifizierungsmitteln.

12. Verwendung der Polyharnstoff- und/oder Polyurethan-Polyorganosiloxan-Verbindungen nach einem oder mehreren der Ansprüche 1 bis 4, oder der reaktiven 1- oder 2-Komponenten-Systeme nach den Ansprüchen 10 oder 11 sowie den gehärteten Zusammensetzungen daraus zur Herstellung von Viskositätsreglern, antistatischen Mitteln, von Mischungskomponenten für Silikon-kautschuke, die peroxidisch oder durch Hydrosilylierung (Platin- Katalyse) zu Elastomeren vernetzt werden können, und dort zur Modifizierung von Oberflächeneigenschaften, zur Modifizierung der Diffusion von Gasen, Flüssigkeiten etc. führen, bzw. das Quell-verhalten der Silikonelastomere modifizieren, in Weichmachern für Textilfasern, zur Behandlung der Textifasern vor, während und nach der Wäsche, in Mitteln zur Modifizierung von natürlichen und synthetischen Fasern, wie beispielsweise Haaren, Baumwollfasern und Synthesefasern, wie Polyesterfasern und Polyamidfasern sowie Mischgeweben, von Textilausrüstungsmitteln, in detergenzienhaltigen Formulierungen, wie in auf anionischen, nichtionogenen und/oder kationischen Tensiden beruhenden Waschmitteln und Reinigungs-mitteln, speziell in festen oder flüssigen Waschmittelformulierungen in Anteilen von etwa 0,1 bis 10 Gew.-% bezogen auf die Gesamtmenge der Fomulierung, in kosmetischen Formulierungen und Faserbehand-lungsformulierungen, wie Textilpflegemitteln, in Anteilen von etwa 0,1 bis 50 Gew.-% bezogen auf die Gesamtmenge der Fomulierung.

13. Verwendung der Polyharnstoff- und/oder Polyurethan-Polyorganosiloxan-Verbindungen nach einem oder mehreren der Ansprüche 1 bis 4, oder der reaktiven 1- oder 2-Komponentensysteme nach den Ansprüchen 10 oder 11 sowie den gehärteten Zusammensetzungen daraus zur Behandlung und Ausrüstung von harten Oberflächen, wie Glas, Keramik, Kacheln, Kunststoffober-flächen, Metalloberflächen, Lackoberflächen, speziell Schiffskörpern und Automobilkarosserien, ganz speziell auch in Trocknerformulierungen für die maschinelle Autowäsche, als Kleber bzw. Primer, bevorzugt zur Verbindung von Siliconenelastomeren mit anderen Substraten, wie Stahl, Aluminium, Glas, Epoxidharz, Polyamid, als Modifikatoren, z.B. Tieftemperaturschlagzäh-Modifikatoren, und Polaritätsmodifikatoren, für kohlenwasserstoffbasierte Polymere und siliconbasierte Elastomersysteme, die auf peroxidischer und Pt katalysierter Vernetzung beruhen.

14. Verwendung der Polyharnstoff- und/oder Polyurethan-Polyorganosiloxan-Verbindungen nach einem oder mehreren der Ansprüche 1 bis 4, oder der reaktiven 1- oder 2-Komponenten-Systeme nach den Ansprüchen 10 oder 11 sowie den gehärteten Zusammensetzungen daraus zur Behandlung von natürlichen und synthetischen Fasern, beispielsweise Baumwolle, Wolle, polyester- und polyamidbasierten Synthesefasern, speziell in Form von Textilien, in speziellen Mitteln zur Faserbehandlung, insbesondere in anionische, nichtionische und/oder kationische Tenside enthaltenden Waschmittelformulierungen, wobei die erfindungsgemäßen Verbindungen in das Waschmittel direkt eingebaut sein können, separat in den laufenden Waschprozeß oder nach dem Waschprozeß dosiert werden können, als Bestandteil separater Weichmachersysteme, speziell auf Basis kationischer Tenside, nach der Wäsche von Fasern und Textilien, als Bügelhilfe und Mittel zur Verhinderung bzw. Rückgängigmachung von Textilverknitterungen, zur Faserausrüstung, speziell zur Erstausrüstung und Behandlung von beispielsweise Baumwolle, Wolle, polyester- und polyamidbasierten Synthesefasern, speziell in Form von Textilien, Papier und Holz.

15. Verwendung der Polyharnstoff- und/oder Polyurethan-Polyorganosiloxan-Verbindungen nach einem oder mehreren der Ansprüche 1 bis 4, oder der reaktiven 1- oder 2-Komponenten-Systeme nach den Ansprüchen 10 oder 11 sowie den gehärteten Zusammensetzungen daraus in kosmetischen Systemen zur Behandlung von Haaren und Haut, bevorzugt als Reinsubstanz, Lösung, Mischung, Emulsion oder Mikroemulsion in Form von Flüssigkeiten, Cremes oder Pasten unterschiedlicher Viskosität, in kosmetischen Formulierungen zur Behandlung keratinhaltiger Substrate wie z. B. menschliche und tierische Haare oder Haut als alkoholische oder polyalkoholische Lösung oder als klare, trübe, weiße Emulsion oder Mikroemulsion, beispielsweise in sogenannten "Rinse-off"-Mitteln wie z.B. "2-in-1"-Shampoos, "Body Wash" und Haarspülungen zur Behandlung von Haar während und nach der Reinigung oder nach dem Färben oder der Behandlung von Haar vor der Bleichung, der Lockung oder Entkräuselung, sowie in sogenannten "Leave-in"-Mitteln wie Haarkuren, Pflegecremes, Frisiercremes, Haargelen, Haarstylingprodukten, Haarfestigern, Haarsprays, Pumpsprays, Fönwellmitteln und Fönfestiger, weiterhin in permanenten, semipermanenten und temporären Haarfärbemitteln, in Mitteln zur Erhöhung des Volumens, der Kämmbarkeit und des Glanzes, sowie der Verminderung des Auswaschens der Farbe von und aus gefärbten keratinhaltigen Substraten wie z.B. menschlichem und tierischem Haar.

## Claims

1. Polyurea- and/or polyurethane-polyorganosiloxane compounds a) containing at least one structural element of the formula (1): 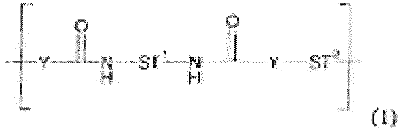 wherein
ST¹ and ST² are each independently selected from divalent or polyvalent, straight-chain, cyclic or branched, saturated, unsaturated or aromatic, substituted or unsubstituted hydrocarbon radicals having up to 1,000 carbon atoms (wherein the carbon atoms of an optionally contained polyorganosiloxane unit are not included), which may contain one or more groups selected from -O-, -NH-, -NR³-, 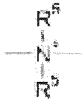 and a polydiorganosiloxane unit having 2 to 1,000 silicon atoms, wherein
R³ is a straight-chain, cyclic or branched, saturated, unsaturated or aromatic hydrocarbon radical having up to 40 carbon atoms, which may contain one or more groups selected from -O-, -C(O)- and -NH-, and
R⁵ is a straight-chain, cyclic or branched, saturated, unsaturated or aromatic hydrocarbon radical having up to 100 carbon atoms, which may contain one or more groups selected from -O-, -C(O)- and -NH-, or R⁵ is a divalent radical which forms cyclic structures within the radicals ST¹ and/or ST²,
and wherein if a plurality of radicals ST¹ is present, these may be identical or different from one another, and if a plurality of radicals ST² is present, these may be identical or different from one another,
with the proviso that
at least one of the radicals ST¹ contains a polyorganosiloxane radical und at least one of the radicals ST² contains a polyalkylene oxide radical, or
at least one of the radicals ST² contains a polyorganosiloxane radical und at least one of the radicals ST¹ contains a polyalkylene oxide radical,
or
polyurea- and/or polyurethane-polyorganosiloxane compounds b) containing at least one structural element of the formula (3): 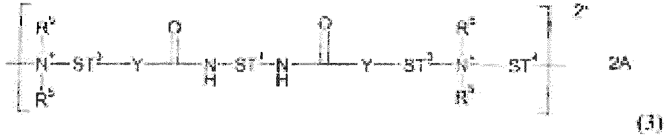 wherein
ST¹ is selected from divalent or polyvalent, straight-chain, cyclic or branched, saturated, unsaturated or aromatic, substituted or unsubstituted hydrocarbon radicals having up to 1,000 carbon atoms (wherein the carbon atoms of an optionally contained polyorganosiloxane unit are not included), which may contain one or more groups selected from -O-, -NH-, -NR³-, 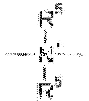 and a polydiorganosiloxane unit having 2 to 1,000 silicon atoms,
wherein R³ and R⁵ are as defined above, und
ST³ is a straight-chain or cyclic or branched, saturated or unsaturated or aromatic, substituted or unsubstituted hydrocarbon radical having 2 to 100 carbon atoms, which may be substituted with -O-, -NH-, -NR³-, wherein R³ is as defined above, and
ST⁴ is a straight-chain or cyclic or branched, saturated or unsaturated or aromatic, substituted or unsubstituted hydrocarbon radical having 2 to 100 carbon atoms (wherein the carbon atoms of an optionally contained polyorganosiloxane unit are not included), which may be substituted with -O-, - NH-, -NR³- and with a polydiorganosiloxane unit having 2 to 200 silicon atoms, wherein R³ is as defined above, and
A⁻ is an organic or inorganic anion,
with the proviso that at least one of the radicals ST¹, ST⁴ contains a polydiorganosiloxane radical, and
wherein the polyurea- and/or polyurethane-polyorganosiloxane compounds b) are obtained by reacting a compound of the formula 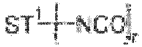 wherein ST¹ is as defined above, with a compound of the formula
HY-ST³-NR⁵₂.
wherein Y, ST³ and R⁵ are as defined above,
and a subsequent reaction with a compound of the formula
Q-ST^{4V}-Q,
wherein Q is a radical which is capable of alkylation of an amino group, and ST^{4V}, together with the molecular part originating from Q after the quaternization reaction, forms the radical ST⁴
Y is -NR²- or -O-, wherein
R² is hydrogen or a straight-chain, cyclic or branched, saturated, unsaturated or aromatic hydrocarbon radical having up to 40 carbon atoms, which may contain one or more groups selected from -O-, -C(O)-, -NH- and -NR³-, wherein R³ is as defined above,
or acid addition compounds and/or salts thereof.

2. The polyurea- and/or polyurethane-polyorganosiloxane compounds according to claim 1, wherein the radicals ST¹, ST², ST³ and ST⁴ are divalent radicals so that the polyurea- and/or polyurethane-polyorganosiloxane compounds are linear in structure.

3. The polyurea-polyorganosiloxane compounds according to claim 1 or 2, wherein Y is NH or NR², wherein R² is as defined above.

4. The polyurea- and/or polyurethane-polyorganosiloxane compounds according to one or more of claims 1 to 3 containing at least one polyalkylene oxide radical.

5. A process for preparing the polyurea- and/or polyurethane-polyorganosiloxane compounds according to one or more of claims 1 to 4, which comprises the reaction of a compound according to the formula: 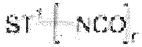 wherein ST¹ is as defined above and r is ≥ 2, with a compound of the formula 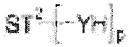 wherein ST² is as defined above and p is ≥ 2, with the proviso that
the polyurea- and/or polyurethane-polyorganosiloxane compounds comprise at least one polyorganosiloxane radical und at least one polyalkylene oxide radical and, in particular, the radical ST¹ contains a polyorganosiloxane radical and the radical ST² contains a polyalkylene oxide radical, or
the radical ST² contains a polyorganosiloxane radical and the radical ST¹ contains a polyalkylene oxide radical.

6. The process for preparing the polyurea- and/or polyurethane-polyorganosiloxane compounds according to claim 5, wherein the compound according to the formula: 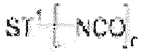 is selected from
aliphatic, di- or polyvalent, straight-chain or branched polyisocyanates having up to 15 carbon atoms, such as hexamethylene diisocyanate, aliphatic, di- or polyvalent, cyclic polyisocyanates having up to 15 carbon atoms, such as 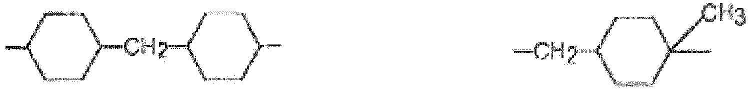 or 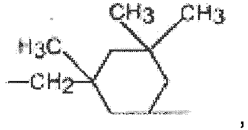 di- or polyvalent, aromatic polyisocyanates having up to 15 carbon atoms, for example based on 2,4-toluyl, 2,6-toluyl, bis-phenylmethane and naphthylene structures, such as 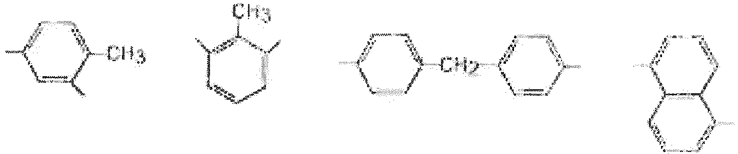 more complex polyisocyanates, such as
for example, isocyanate-terminated polyethers, preferably ethylene oxide- and propylene oxide-based polyethers, for example prepared from primary amino-terminated polyethers of the Jeffamine® type, for example of the ED- and T-series (Huntsman Corp.),
isocyanate-terminated polyamides,
isocyanate-terminated polyureas,
isocyanate-terminated polyurethanes,
isocyanatoalkyl-functionalized polyorganosiloxanes, such as polyisocyanates of higher functionality which are derived from amino group containing polyorganosiloxanes, wherein the primary amino functions are arranged comb-like and optionally in the α,ω,-position.

7. The process for preparing the polyurea- and/or polyurethane-polyorganosiloxane compounds according to claims 5 or 6, wherein the compound according to the formula: 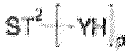 is selected from
aliphatic, di- or polyvalent, straight-chain or branched polyols or polyamines having up to 15 carbon atoms, such as hexamethylenediamine or -diol, aliphatic, di- or polyvalent, cyclic polyols or polyamines having up to 15 carbon atoms, for example based on biscyclohexylmethane structures 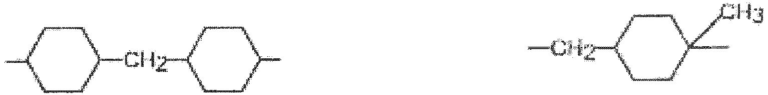 or 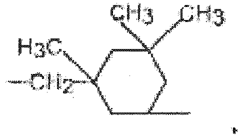 di- or polyvalent, aromatic polyols or polyamines having up to 15 carbon atoms, for example based on 2,4-toluyl, 2,6-toluyl, bis-phenylmethane and naphthylene structures, such as 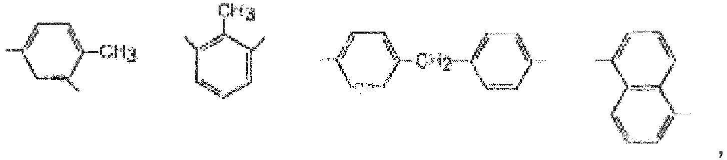 in particular, hexamethylenediamine, phenylenediamine, toluenediamines, cyclohexanediamine, ethylenediamine, propylenediamine,
divalent or polyvalent primary and/or secondary amines or alcohols, such as, for example, N(CH₂CH₂NH₂)₃, diethylenetriamine, dipropylenetriamine and higher oligomers, glycerol and alkoxylated derivatives thereof,
more complex divalent or more than divalent polyamines or polyols, such as primary and secondary amino- or hydroxyl-functionalized prepolymers, which may likewise be of higher functionality, such as, for example,
- primary or secondary amino- or hydroxyl-terminated polyethers, preferably ethylene oxide- and propylene oxide-based polyethers, for example primary or secondary amino-terminated polyethers of the Jeffamine® type, for example of the ED- and T-series (Huntsman Corp.),
- NH₂-terminated polyamides,
- NH₂-terminated polyureas,
- NH₂-terminated polyurethanes,
- OH-terminated polyureas,
- OH-terminated polyurethanes,
- OH-terminated polyesters,
- α,ω,-primary or secondary amino- or hydroxyalkyl- or hydroxypolyetheralkyl-functionalized polyorganosiloxanes, such as primary or secondary amino or hydroxyalkyl or hydroxypolyetheralkyl groups containing polyorganosiloxanes of higher functionality, wherein the amino or hydroxyl functions are arranged comb-like and optionally in the α,ω-position.

8. A process for preparing the polyurea- and/or polyurethane-polyorganosiloxane compounds according to one or more of claims 1 to 4, according to formula (3): 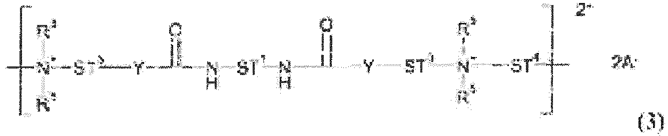 wherein A⁻, R⁵, ST¹, ST², ST³, ST⁴ and Y are as defined above, the process comprising the reaction of a compound of the formula: 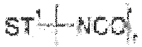 wherein ST¹ is as defined above, with a compound of the formula:
HY-ST³-NR³₂.
wherein Y, ST³ and R⁵ are as defined above,
and a subsequent reaction with a compound of the formula:
Q-ST^{4V}-Q.
wherein Q is a radical which is capable of alkylation of an amino group, and ST^{4V}, together with the molecular part originating from Q after the quaternization reaction, forms the radical ST⁴.

9. A reactive 1- or 2-component system containing at least one compound of the formula 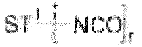 wherein ST¹ is as defined above and r is ≥ 2, wherein the isocyanate groups can optionally be blocked in a manner known per se,
and at least one compound of the formula 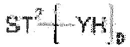 wherein ST² is as defined above and p is ≥ 2, with the proviso that
the radical ST¹ contains a polyorganosiloxane radical and
the radical ST² contains a polyalkylene oxide radical, or
the radical ST² contains a polyorganosiloxane radical and
the radical ST¹ contains a polyalkylene oxide radical,
and optionally fillers, pigments, further binders, auxiliary substances, solvents.

10. A reactive 1- or 2-component system containing at least one compound of the formula 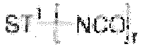 wherein ST¹ is as defined above, and at least one compound of the formula
HY-ST³-_{NR}⁵₂.
wherein Y, ST³ and R⁵ are as defined above,
and at least one compound of the formula
Q-ST^{4V}-Q,
wherein Q is a radical which is capable of alkylation of an amino group, and ST^{4V} together with the molecular part originating from Q after the quaternization reaction, forms the radical ST⁴,
and optionally fillers, pigments, further binders, auxiliary substances, solvents.

11. Use of the polyurea- and/or polyurethane-polyorganosiloxane compounds according to one or more of claims 1 to 4 or the reactive 1- or 2-component systems of claims 9 or 10, as well as cured compositions thereof, for the preparation of coatings, agents for modification of surfaces, elastomers, thermosets, adhesives, primers for metal and plastics surfaces, polymer additives, detergent additives, rheological agents, cosmetic products, and agents for modification of fibres.

12. Use of the polyurea- and/or polyurethane-polyorganosiloxane compounds according to one or more of claims 1 to 4 or the reactive 1- or 2-component systems of claims 10 or 11, as well as cured compositions thereof, for the preparation of viscosity regulators, antistatic agents, mixing components for silicone rubbers which can be crosslinked to elastomers peroxidically or by hydrosilylation (platinum catalysis), and lead to a modification of the surface properties, to a modification of the diffusion of gases, liquids etc. therein, or modify the swelling properties of the silicone elastomers, in softeners for textile fibres, for treatment of the textile fibres before, during and after washing, in compositions for modification of natural and synthetic fibres, such as, for example, hair, cotton fibres and synthetic fibres, such as polyester fibres and polyamide fibres, as well as blended fabrics, of textile finishing compositions, in detergent-containing formulations, such as in detergents and cleaning compositions based on anionic, nonionic and/or cationic surfactants, specifically in solid or liquid detergent formulations in contents of from about 0.1 to 10 wt. %, based on the total amount of the formulation, in cosmetic formulations and fibre treatment formulations, such as to textile care compositions, in contents of from about 0.1 to 50 wt. %, based on the total amount of the formulation.

13. Use of the polyurea- and/or polyurethane-polyorganosiloxane compounds according to one or more of claims 1 to 4 or the reactive 1- or 2-component systems of claims 10 or 11, as well as cured compositions thereof, for the treatment and finishing of hard surfaces, such as glass, ceramic, tiles, plastics surfaces, metal surfaces, lacquer surfaces, specifically ship hulls and automobile bodies, quite specifically also in dryer formulations for mechanical washing of automobiles, as adhesives or primers, preferably for bonding silicone elastomers with other substrates, such as steel, aluminum, glass, epoxy resin, polyamide, as modifiers, e.g. low temperature impact modifiers and polarity modifiers, for hydrocarbon-based polymers and silicone-based elastomer systems based on peroxidic and Pt-catalysed crosslinking.

14. Use of the polyurea- and/or polyurethane-polyorganosiloxane compounds according to one or more of claims 1 to 4 or the reactive 1- or 2-component systems of claims 10 or 11, as well as cured compositions thereof, for treatment of natural and synthetic fibres, for example cotton, wool, polyester- and polyamide-based synthetic fibres, specifically in the form of textiles, in specific compositions for fibre treatment, in particular in detergent formulations containing anionic, nonionic and/or cationic surfactants, wherein the compounds according to the invention can be incorporated directly into the detergent or can be metered in separately as the washing process runs or after the washing process, as a constituent of separate softener systems, specifically based on cationic surfactants, after washing of fibres and textiles, as an ironing aid and an agent for preventing or reversing creases in textiles, for finishing fibres, specifically for the first finishing and treatment of, for example, cotton, wool, polyester- and polyamide-based synthetic fibres, specifically in the form of textiles, paper and wood.

15. Use of the polyurea- and/or polyurethane-polyorganosiloxane compounds according to one or more of claims 1 to 4 or the reactive 1- or 2-component systems of claims 10 or 11, as well as cured compositions thereof, in cosmetic systems for the treatment of hair and skin, preferably as the pure substance, solution, mixture, emulsion or microemulsion in the form of liquids, creams or pastes of differing viscosity, in cosmetic formulations for treatment of keratin-containing substrates, such as e.g. human and animal hair or skin, as an alcoholic or polyalcoholic solution or as a clear, cloudy, white emulsion or microemulsion, for example in so-called "rinse-off" compositions, such as e.g. "2-in-1" shampoos, "body wash" formulations and hair rinses for treatment of hair during and after cleansing or after colouring or the treatment of hair before bleaching, curling or straightening, and in so-called "leave-in" compositions, such as hair treatments, care creams, hairdressing creams, hair gels, hair styling products, hair setting compositions, hair sprays, pump sprays, blow-waving compositions and blow-drying compositions, furthermore in permanent, semi-permanent and temporary hair colouring compositions, in compositions for increasing the volume, the combability and the shine, and for reducing washing out of the colour from and out of coloured keratin-containing substrates, such as e.g. human and animal hair.

## Revendications

1. Composés de polyurée et/ou de polyuréthane-polyorganosiloxane a), contenant au moins un élément de structure de la formule (1): 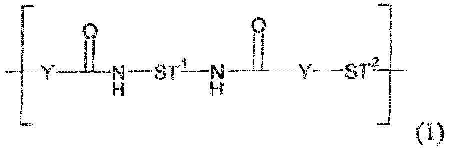 dans laquelle
ST¹ et ST² sont sélectionnés indépendamment l'un de l'autre parmi les résidus hydrocarbonés bivalents ou supérieurs, à chaîne droite, cycliques ou ramifiés, saturés, insaturés ou aromatiques, substitués ou non-substitués ayant jusqu'à 1000 atomes de carbone (les atomes de carbone d'une unité de polyorganosiloxane éventuellement contenue dans ceux-ci n'étant pas inclus), pouvant contenir un ou plusieurs groupes, sélectionnés parmi
-O-, -NH-, NR³- 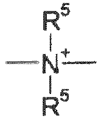 et une unité de polydiorganosiloxane ayant 2 à 1000 atomes de silicium, dans laquelle
R³ est un résidu hydrocarboné à chaîne droite, cyclique ou ramifié, saturé, insaturé ou aromatique, ayant jusqu'à 40 atomes de carbone, pouvant contenir un ou plusieurs groupes, sélectionnés parmi -O-, --C(O)- et -NH-, et,
R⁵ est un résidu hydrocarboné à chaîne droite, cyclique ou ramifié, saturé, insaturé ou aromatique ayant jusqu'à 100 atomes de carbone, pouvant contenir un ou plusieurs groupes, sélectionnés parmi -O-, -C(O) -et NH-, ou R⁵ est un résidu bivalent, formant des structures cycliques au sein des résidus ST¹ et/ou ST²_{,}
et dans laquelle, lorsque une pluralité de résidus ST¹ est présente, ceux-ci peuvent être égaux ou différents l'un de l'autre, et lorsque une pluralité de résidus ST² est présente, ceux-ci peuvent être égaux ou différents l'un de l'autre,
à condition que
au moins l'un des résidus ST¹ comprend un résidu de polyorganosiloxane et au moins l'un des résidus ST² comprend un résidu de polyalkylènoxyde,
ou
au moins l'un des résidus ST² comprend un résidu de polyorganosiloxane et au moins l'un des résidus ST¹ comprend un résidu de polyalkylènoxyde.
ou
Composés de polyurée et/ou de polyuréthane-polyorganosiloxane b), contenant au moins un élément de structure de la formule (3): 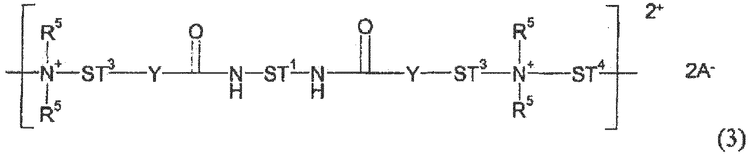 dans laquelle
ST¹ est sélectionné parmi les résidus hydrocarbonés bivalents ou supérieurs, à chaîne droite, cycliques ou ramifiés, saturés, insaturés ou aromatiques, substitués ou non-substitués ayant jusqu'à 1000 atomes de carbone (les atomes de carbone d'une unité de polyorganosiloxane éventuellement contenue dans ceux-ci n'étant pas inclus), pouvant contenir un ou plusieurs groupes, sélectionnés parmi sélectionnés parmi
-O-, -NH-, NR³- 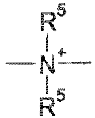 et une unité de polydiorganosiloxane ayant 2 à 1000 atomes de silicium,
dans laquelle R³ et R⁵ sont tel que définis au-dessus, et
ST³ est un résidu hydrocarboné à chaîne droite ou cyclique ou ramifié, saturé ou insaturé ou aromatique, substitué ou non-substitué ayant 2 à 100 atomes de carbone, pouvant être substitué par -O-,-NH-, -NR³-, dans laquelle R³ est tel que défini au-dessus, et
ST⁴ est un résidu hydrocarboné à chaîne droite ou cyclique ou ramifié, saturé ou insaturé ou aromatique, substitué ou non-substitué ayant 2 à 100 atomes de carbone (les atomes de carbone d'une unité de polyorganosiloxane éventuellement contenue dans ceux-ci n'étant pas inclus), pouvant être substitué par -O-,-NH-, -NR³- et par une unité de polydiorganosiloxane ayant 2 à 200 atomes de silicium, dans laquelle R³ est tel que défini au-dessus, et
A⁻ est un anion organique ou inorganique,
à condition que au moins l'un des résidus ST¹, ST⁴ contient un résidu de polydiorganosiloxane,
et dans laquelle les composés de polyurée et/ou de polyuréthane-polyorganosiloxane b) sont obtenus par la réaction d'un composé de la formule 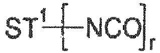 dans laquelle ST¹ est tel que défini au-dessus, avec un composé de la formule
HY-ST³ -NR⁵₂,
dans laquelle Y, ST³ et R⁵ sont tel que définis au-dessus, et après avec un composé de la formule
Q-ST^{4V}-Q,
dans laquelle Q est un résidu capable d'alkylation d'un groupe amino, et ST^{4V} ensemble avec la partie de la molécule résultant de Q après une réaction de quatérnisation, forme le résidu ST⁴
Y est -NR²- ou -O-, dans lequel
R² est de l'hydrogène ou un résidu hydrocarboné à chaîne droite, cyclique ou ramifié, saturé, insaturé ou aromatique, ayant jusqu'à 40 atomes de carbone, pouvant contenir un ou plusieurs groupes, sélectionnés parmi -O-, -C(O)-,-NH- et -NR³-, dans lequel R³ est tel que défini au-dessus,
ou les composés d'addition des acides et/ou les sels de ceux-ci.

2. Composés de polyurée et/ou de polyuréthane-polyorganosiloxane selon la revendication 1, dans lequel les résidus ST¹, ST², ST3 et ST⁴ sont les résidus bivalents, afin que les composés de polyurée et/ou de polyuréthane-polyorganosiloxane soient constitués de façon linéaire.

3. Composés de polyuréthane-polyorganosiloxane selon la revendication 1 ou 2, dans lequel Y est: NH ou NR², dans lequel R² est tel que défini au-dessus.

4. Composés de polyurée et/ou de polyuréthane-polyorganosiloxane selon l'une ou plusieurs des revendications 1 à 3, contenant au moins un résidu de polyalkylènoxyde.

5. Procédé de fabrication de composés de polyurée et/ou de polyuréthane-polyorganosiloxane selon l'une ou plusieurs des revendications 1 à 4, comprenant la réaction d'un composé de la formule: 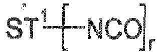 dans lequel ST¹ est tel que défini au-dessus, et r est ≥ 2, avec un composé de la formule 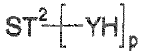 dans lequel ST² est tel que défini au-dessus, et p est ≥ 2, à condition que
les composés de polyurée et/ou de polyuréthane-polyorganosiloxane comprennent au moins un résidu de polyorganosiloxane et au moins un résidu de polyalkylènoxyde, et particulièrement le résidu ST¹ comprend un résidu de polyorganosiloxane et le résidu ST² comprend un résidu de polyalkylènoxyde, ou
le résidu ST2 comprend un résidu de polyorganosiloxane et le résidu ST¹ comprend un résidu de polyalkylènoxyde.

6. Procédé de fabrication de composés de polyurée et/ou de polyuréthane-polyorganosiloxane selon la revendication 5, dans lequel le composé de la formule 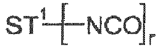 est sélectionné parmi
les polyisocyanates aliphatiques, bivalents ou polyvalents, à chaîne droite ou ramifiés ayant jusqu'à 15 atomes de carbone,
par exemple hexamethylènediisocyanat, les polyisocyanates aliphatiques, bivalents ou polyvalents, cycliques ayant jusqu'à 15 atomes de carbone, tel que par exemple 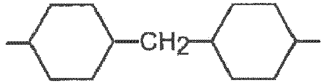 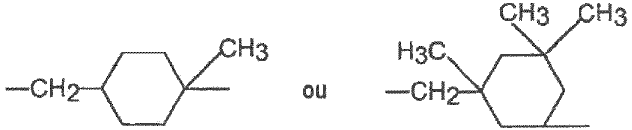 les polyisocyanates bivalents ou polyvalents, aromatiques ayant jusqu'à 15 atomes de carbone,
par exemple à base de structures de 2,4-toluyle, 2,6-toluyle, diphényle méthane et naphtylène, tel que 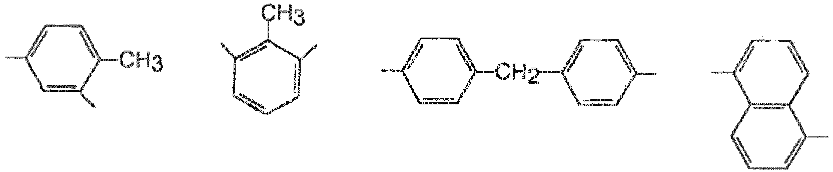 les polyisocyanates plus complexes,
tel que par exemple le polyéther terminé en isocyanate, préférablement le polyéther à base d'oxyde d'éthylène et propylène, par exemple fabriqué de polyéthers primaires terminés en amino du type Jeffamine®,
par exemple de la série ED et T (Huntsman Corp.),
les polyamides terminés en isocyanate,
les polyurées terminées en isocyanate,
les polyuréthanes terminés en isocyanate,
les polyorganosiloxanes à fonctionnalité isocyanatoalkyle, tel que les polyisocyanates à fonctionnalité supérieure, qui dérivent des polyorganosiloxanes portant des groupes amino primaires ayant des fonctions amino primaires en forme d'une peigne, et le cas échéant, situés dans une position a,w.

7. Procédé de fabrication de composés de polyurée et/ou de polyuréthane-polyorganosiloxane selon l'une des revendications 5 ou 6, dans lequel le composé de la formule: 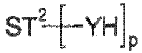 est sélectionné parmi
les polyols ou polyamines aliphatiques, bivalents ou polyvalents, à chaîne droite ou ramifiés ayant jusqu'à 15 atomes de carbone, par exemple le hexaméthylènediamine ou le hexaméthylènediol,
les polyols ou les polyamines aliphatiques, bivalentes ou polyvalentes, cycliques ayant jusqu'à 15 atomes de carbone, par exemple à base de structures de biscyclohexylméthane 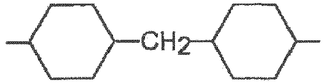 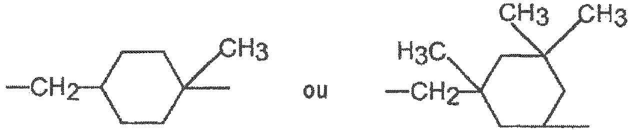 les polyols ou les polyamines bivalents ou polyvalents, aromatiques ayant jusqu'à 15 atomes de carbone, par exemple à base de structures de 2,4-toluyle, 2,6-toluyle, diphényle méthane et naphtylène, 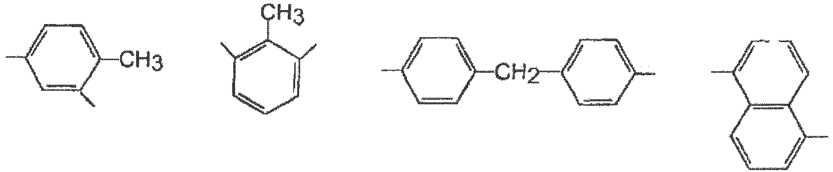 particulièrement l'hexamethylènediamin, la phenylènediamine, la toluènediamine, la cyclohéxanediamine, l'éthylènediamine, la propylènediamine,
les amines ou les alcools secondaires ou primaires bivalents ou supérieurs, tel que par exemple N(CH₂CH₂NH₂)₃, la diethylènetriamin, la dipropylènetriamine et les oligomères supérieurs, la glycérine et ses dérivés alkoxylés,
les polyols ou les polyamines plus complexes, bivalents ou supérieurs, qui également peuvent être à fonctionnalité supérieure, tel que par exemple
- les polyéthers primaires ou secondaires terminés en amino ou hydroxyle, préférablement les polyéthers à base d'oxyde d'éthylène et d'oxyde de propylène, par exemple les polyéther primaires ou secondaires terminés en amino du type Jeffamine®,
- par exemple de la série ED et T (Huntsman Corp.)
- les polyamides terminés en NH₂,
- les polyurées terminés en NH₂,
- les polyuréthanes terminés en NH₂,
- les polyurées terminés en OH,
- les polyuréthanes terminés en OH,
- les polyesters terminés en OH,
- les polyorganosiloxanes à fonctionnalité amino ou hydroxyalkyle ou hydroxypolyétheralkyle primaire ou secondaire en α,ω, tel que les polyorganosiloxanes à fonctionnalité supérieure portant des groupes amino primaires ou secondaires ou des groupes hydroxyalkyle ou des groupes hydroxypolyétheralkyle qui éventuellement ont des fonctions amino ou hydroxyle dans la position α,ω.

8. Procédé de fabrication de composés de polyurée et/ou de polyuréthane-polyorganosiloxane selon l'une ou plusieurs des revendications 1 à 4, de la formule (3) 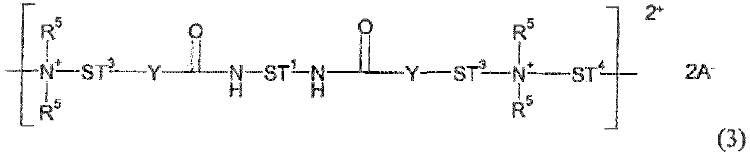 dans laquelle
A⁻, R⁵, ST¹, ST³ ST⁴ et Y sont tel que définis au-dessus, qui comprend la réaction d'un composé de la formule 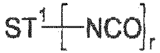 dans laquelle ST¹ est tel que défini au-dessus, avec un composé de la formule
HY-ST³-N R⁵₂,
dans laquelle Y, ST³ et R⁵ sont tel que définis au-dessus,
et ensuite avec un composé de la formule
Q-ST^{4V}-Q,
dans laquelle Q est un résidu capable d'alkylation d'un groupe amino, et ST^{4V}, ensemble avec la partie de la molécule résultant de Q après une réaction de quatérnisation, forme le résidu ST⁴.

9. Système réactif à 1 ou 2 composants, contenant au moins un composé de la formule, 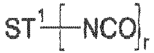 dans laquelle ST¹ est tel que défini en-dessus, et r est ≥ 2, dans laquelle les groupes isocyanate, le cas échéant, peuvent être bloqués d'une façon connue en soi,
et au moins un composé de la formule 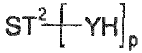 dans laquelle ST² est tel que défini au-dessus, et est p > 2, à condition que
le résidu ST¹ comprend un résidu de polyorganosiloxane et
le résidu ST² comprend un résidu de polyalkylènoxyde, ou
le résidu ST² comprend un résidu de polyorganosiloxane et
le résidu ST¹ comprend un résidu de polyalkylènoxyde,
ainsi que, le cas échéant, les charges, les pigments, d'autres liants, additifs, solvants.

10. Système réactif à 1 ou 2 composants, contenant au moins un produit de réaction à partir d'au moins un composé de la formule 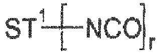 dans laquelle ST¹ est tel que défini au-dessus, et d'au moins un composé de la formule
HY-ST³-NR⁵₂,
dans laquelle Y, ST³ et R⁵ sont tel que définis au-dessus,
et d'au moins un composé de la formule
Q-ST^{4V}-_{Q}
dans laquelle Q est un résidu capable d'alkylation d'un groupe amino,
et ST^{4v}, ensemble avec la partie de la molécule résultant de Q après une réaction de quatérnisation, forme le résidu ST⁴,
ainsi que, le cas échéant, les charges, les pigments, d'autres liants, additifs, solvants.

11. Utilisation des composés de polyurée et/ou de polyuréthane-polyorganosiloxane selon l'une ou plusieurs des revendications 1 à 4, ou des systèmes réactifs à 1 ou 2 composants selon les revendications 9 ou 10 ainsi que des compositions durcies à partir de ceux-ci pour la fabrication des revêtements, agents pour la modification des surfaces, duromères, adhésives, primaires pour les surfaces métalliques et plastiques, additifs polymères, additifs détergents, agents rhéologiques, agents cosmétiques, agents modifiant les fibres.

12. Utilisation des composés de polyurée et/ou de polyuréthane-polyorganosiloxane selon l'une ou plusieurs des revendications 1 à 4, ou des systèmes réactifs à 1 ou 2 composants selon les revendications 10 ou 11 ainsi que des compositions durcies à partir de ceux-ci pour la fabrication des régulateurs de viscosité, agents antistatiques, composants de mélange pour les caoutchoucs de silicone, qui peuvent être réticulés au peroxyde ou par hydrosilylation (catalyse de platine) aux élastomères, tout en résultant à la modification des propriétés de surface, la modification de la diffusion de gazes, liquides etc., modifiant le gonflement des élastomères de silicone, respectivement, dans les adoucissants pour les fibres textiles, pour le traitement des fibres textiles avant, pendant et après le lavage, dans les agents pour la modification des fibres naturels et synthétiques, tel que par exemple les cheveux, les fibres de coton et les fibres synthétiques, tel que les fibres de polyester et les fibres de polyamide, ainsi que les tissus mélangés, des produits auxiliaires textiles, dans les formulations contenant de détergents, tel que les produits de lavage et de nettoyage à base de tensioactifs anioniques, non-ionogènes et/ou de tensioactifs cationiques, notamment dans les formulations de produit de lavage solides et liquides dans les proportions d'environ 0,1 à 10 % en poids relatif à la quantité totale de la formulation, dans les formulations cosmétiques et les formulations pour le traitement des fibres, tel que les produits de soins pour textiles, dans les proportions d'environ 0,1 à 50 % en poids relatif à la quantité totale de la formulation.

13. Utilisation des composés de polyurée et/ou de polyuréthane-polyorganosiloxane selon l'une ou plusieurs des revendications 1 à 4, ou des systèmes réactifs à 1 ou 2 composants selon les revendications 10 ou 11 ainsi que des compositions durcies à partir de ceux-ci pour le traitement et l'apprêt des surfaces durs, tel que le verre, la céramique, les tuiles, les surfaces en matière plastique, les surfaces métalliques et les surfaces de vernis, notamment les corps de navire, et les carrosseries d'automobile, plus spécifiquement aussi dans les formulations de séchage pour le lavage des voitures par machine, à titre d'adhésive et de primaire, respectivement, préférablement pour la combinaison d'élastomères de silicone avec d'autres substrats, tel que l'acier, l'aluminium, le verre, le résine d'époxyde, le polyamide, à titre de modificateurs, par exemple les modificateurs d'impact à température basse et les modificateur de la polarité pour les polymères hydrocarbonées et les systèmes élastomères siliconés qui sont basés sur la réticulation au peroxyde et à catalyse platine.

14. Utilisation des composés de polyurée et/ou de polyuréthane-polyorganosiloxane selon l'une ou plusieurs des revendications 1 à 4, ou des systèmes réactifs à 1 ou 2 composants selon les revendications 10 ou 11 ainsi que des compositions durcies à partir de ceux-ci pour le traitement des fibres naturels et synthétiques , par exemple le coton, la laine, les fibres synthétiques à base de polyester et de polyamide, notamment en forme de textiles, dans les agent spécifiques pour le traitement de fibres, particulièrement les tensioactifs anioniques, non ioniques et/ou cationiques contenant des formulations de produit de lavage, dans lesquelles les composés selon l'invention peuvent être directement incorporés dans la lessive, peuvent être dosés séparément dans le procédé de lavage en cours ou après le procédé de lavage, à titre de composant de systèmes d'adoucissants séparés, notamment à base de tensioactifs cationiques, après le lavage de fibres et textiles, à titre d'aide au repassage et les agents pour l'empêchement et la réversion, respectivement, du froissement de textiles, pour l'apprêt de fibres, notamment pour l'apprêt primaire et le traitement de par exemple coton, laine, fibres synthétiques à base de polyester-et polyamide, notamment en forme de textiles, de papier et de bois.

15. Utilisation des composés de polyurée et/ou de polyuréthane-polyorganosiloxane selon l'une ou plusieurs des revendications 1 à 4, ou des systèmes réactifs à 1 ou 2 composants selon les revendications 10 ou 11 ainsi que des compositions durcies à partir de ceux-ci dans les systèmes cosmétiques pour le traitement des cheveux et de la peau, préférablement comme substance pure, solution, mélange, émulsion, ou microémulsion en forme de liquides, crèmes, ou pâtes de viscosité différente, dans les formulations cosmétiques pour le traitement de substrats contenant de la kératine, tel que z. B. les cheveux ou la peau humaine ou des animaux, à tire de solution alcoolique ou polyalcoolique ou à titre d'émulsion ou microémulsion claire, trouble, blanche, par exemple dans les agents dits agents 'Rinse-off', tel que par exemple les « shampooings 2-dans-1", "Body Wash" et les rinçages capillaires pour le traitement de cheveux, pendant ou après le nettoyage ou après la teinture ou le traitement de cheveux avant de blanchir, boucler ou défriser, ainsi que les agents dits agents "Leave-in », tel que les traitements capillaire, les crèmes de soins, les crèmes de coiffage, les gels capillaires, les produits 'hairstyling', les fixatifs pour cheveux, les sprays capillaires, les sprays de pompe, les agents 'brushing' et les fixatifs thermo-actifs afin de sécher au sèche-cheveux, en outre les teintures de cheveux permanentes, semi-permanentes et temporaires, dans les agents pour augmenter la volume, le coiffage et la brillance, ainsi que pour éviter l'élimination de la couleur de et à partir des substrats colorés, contenant de la kératine, tel que par exemple les cheveux humains et des animaux.
